# EUROPEAN PATENT APPLICATION

(11) **EP 4 834 367 A1**
(43) Date of publication of application: **04.11.2026**
(21) Application number: 24912935.4
(22) Date of filing: 25.12.2024
(51) Int. Cl.: A61K 45/00, A61P 25/28

(54) **PROPHYLACTIC AND/OR THERAPEUTIC AGENT FOR NEURODEGENERATIVE DISEASE**

(30) Priority: 27.12.2023 US 202363615036 P
(71) Applicant: RIKEN, Wako-shi, Saitama 351-0198 (JP)
(72) Inventor: FUKUI Masahiro, Wako-shi, Saitama 351-0198 (JP); KAGEYAMA Ryoichiro, Wako-shi, Saitama 351-0198 (JP)
(74) Representative: Mewburn Ellis LLP
(86) International application number: PCT/JP2024/045989
(87) International publication number: WO 2025/143049

(57) **Abstract**

The purpose of the present invention is to provide a novel prophylactic and/or therapeutic agent for a neurodegenerative disease, which can increase the number of new neurons in an adult brain. The present invention is a prophylactic and/or therapeutic agent for a neurodegenerative disease, the agent containing, as an active ingredient, an inhibitor of any one of genes shown in table 1 or a protein derived from any one of the genes.

## Description

### TECHNICAL FIELD

The present invention relates to a novel method for activating neurogenesis and suppressing amyloid *β* deposition in the adult brain, and a prophylactic and/or therapeutic agent for neurodegenerative diseases using this method.

### BACKGROUND ART

It has become clear that neural stem cells and neural progenitor cells exist in adult mammalian brains, including humans, although their absolute numbers are small, and that in some regions of the brain, they carry out neurogenesis. However, the majority of neural stem cells in the adult brain are in a quiescent state and have low proliferative and neurogenic capacities. Since this proliferative and neurogenic capacities further decrease with aging, there are almost no newly born neurons (newborn neurons) in the aged brain. A significant decrease in newborn neurons is also considered to be a cause of decline in brain function such as dementia, as well as neurodegenerative diseases such as Alzheimer's disease and Parkinson's disease.

Under such circumstances, attempts have been made to amplify and differentiate the neural stem cells and neural progenitor cells, which are present in small numbers in the adult brain, through stimulation with growth factors and the like, in order to promote functional recovery (see Non-Patent Literature 1). However, the effect cannot be said to be sufficient. Moreover, research on cell transplantation therapy models using neural stem cells and neural progenitor cells derived from fetal brains, embryonic stem (ES) cells, and iPS cells is also actively being conducted. However, it is still unclear whether normal neural networks can be reconstructed through cell transplantation therapy in the brain, and the risk of side effects is also high. Furthermore, many attempts have been made so far to increase the number of newborn neurons in the adult brain, but their effects are still limited (see Non-Patent Literature 1 to 5).

On the other hand, the inventors of the present application previously demonstrated that aged neural stem cells, which have already lost their proliferative and neurogenetic abilities, can be functionally rejuvenated by inducing the expression of the zinc finger transcription factor gene Plagl2 and inhibiting the activity of Dyrk1a, a gene associated with Down syndrome (a hereditary disease known to accelerate aging). It was demonstrated that such rejuvenated neural stem cells continuously proliferate at levels observed in the juvenile hippocampus, produce new neurons, and improve cognition. It was also found that this treatment activates neurogenesis, reduces A*β* deposition, and improves cognition in 5XFAD mice, model mice of Alzheimer's disease (Non-Patent Literature 6, Patent Literature 1).

### PRIOR ART DOCUMENTS Patent Literature

Patent Literature 1: International Publication No. WO2022/186089

### Non-Patent Literature

Non-Patent Literature 1: Nagahara AH et al. (2009) Neuroprotective effects of brain-derived neurotrophic factor in rodent and primate models of Alzheimer's disease, Nat.Med., vol. 15(3), pp.331-337.
Non-Patent Literature 2: Henriette van Praag et al., Exercise enhances learning and hippocampal neurogenesis in aged mice, J Neurosci., 2005 Sep 21; 25(38): 8680-5
Non-Patent Literature 3: Benedetta Artegiani et al., Overexpression of cdk4 and cyclinD1 triggers greater expansion of neural stem cells in the adult mouse brain. J Exp Med. 2011 May 9; 208(5): 937-48
Non-Patent Literature 4: Benraiss, A.et al. (2013) Mobilization of endogenous progenitor cells regenerates functionally-integrated medium spiny striopallidal projection neurons and delays disease progression in a transgenic model of Huntington's disease. Cell Stem Cell 12, 787-799
Non-Patent Literature 5: Berdugo-Vega, G. et al. (2020). Increasing neurogenesis refines hippocampal activity rejuvenating navigational learning strategies and contextual memory throughout life. Nat. Commun. 11, 135
Non-Patent Literature 6: Kaise, T.et.al., (2022) Functional rejuvenation of aged neural stem cells by Plagl2 and anti-Dyrk1a activity. Genes Dev 36: 23-37

### SUMMARY OF INVENTION

### Technical Problem

The present invention aims to provide a novel method for activating neurogenesis and suppressing amyloid-*β* (A*β*) deposition in the adult brain, and also to provide a prophylactic and/or therapeutic agent for neurodegenerative diseases using this method.

### Solution to Problem

The present inventors previously demonstrated that administration of lentivirus with Plagl2 expression-inducing activity and anti-Dyrk1a (iPaD) activity to 5XFAD mice, model mice of Alzheimer's disease, efficiently activates neurogenesis in the adult brain, reduces amyloid-*β* (A*β*) deposition, and improves cognitive function. Based on these research results, the present inventors have further advanced their research and investigated the downstream events of iPaD treatment in 5XFAD mice. Through global RNA-seq and functional analyses, it has been revealed that iPaD treatment in 5XFAD mice significantly reduces the expression of specific genes and significantly enhances the expression of specific genes (ligand-receptor pairs). Furthermore, when Prkag2 (protein kinase AMP-activated non-catalytic subunit gamma 2), Maml2 (Mastermind2), Gfra2 (glial cell line-derived neurotrophic factor receptor alpha-2), Rpl29 (ribosomal protein L29), among the specific genes above whose expressions were reduced by iPaD treatment, were each knocked down, the obtained results have shown that neurogenesis in the adult brain was efficiently activated and the deposition of amyloid-*β* (A*β*) was reduced. As the activation of neurogenesis in the adult brain has a potential to improve age-related cognitive impairments and neurodegenerative diseases, it has been strongly suggested that knockdown of Prkag2, Gfra2, Maml2, and/or Rpl29 is a promising therapeutic strategy for such neurodegenerative diseases, including Alzheimer's disease.

Specifically, the present invention relates to nucleic acid drugs and the like which target specific genes whose expressions have been found to decrease in the downstream of iPaD treatment, the nucleic acid drugs and the like making it possible to prevent and treat neurodegenerative diseases such as Alzheimer's disease by controlling the expressions of these genes. That is, the gist of the present invention is as follows.
[1] A prophylactic and/or therapeutic agent for neurodegenerative diseases, comprising as an active ingredient an inhibitor of any of the genes listed in Table 1 below or of a protein derived from any of these genes.

**[Table 1]**

| ID | Official Symbol | Gene Name | Ensembl Gene ID | Transcript ID |
|---|---|---|---|---|
| 1 | Maml2 | mastermind like transcriptional coactivator 2 | ENSMUSG00000031925 | ENSMUST00000159294 |
| 2 | Rpl29 | ribosomal protein L29 | ENSMUSG00000048758 | ENSMUST00000150576 |
| 3 | Ptgds | prostaglandin D2 synthase (brain) | ENSMUSG00000015090 | ENSMUST00000015234 |
| 4 | Ell3 | elongation factor RNA polymerase II-like 3 | ENSMUSG00000027246 | ENSMUST00000028679 |
| 5 | Grik3 | glutamate receptor, ionotropic, kainate 3 | ENSMUSG00000001985 | ENSMUST00000030676 |
| 6 | Col5a1 | collagen, type V, alpha 1 | ENSMUSG00000026837 | ENSMUST00000028280 |
| 7 | Prkag2 | protein kinase, AMP-activated, gamma 2 non-catalytic subunit | ENSMUSG00000028944 | ENSMUST00000030784 |
| 8 | Cspp1 | centrosome and spindle pole associated protein 1 | ENSMUSG00000056763 | ENSMUST00000071087 |
| 9 | A830036E02Rik | RIKEN cDNA A830036E02 gene | ENSMUSG00000084890 | ENSMUST00000139095 |
| 10 | 4930474M22Rik | RIKEN cDNA 4930474M22 nene | ENSMUSG00000117328 | ENSMUST00000233310 |
| 11 | Gfra2 | glial cell line derived neurotrophic factor family receptor alpha 2 | ENSMUSG00000022103 | ENSMUST00000022699 |
| 12 | Itpk1 | inositol 1,3,4-triphosphate 5/6 kinase | ENSMUSG00000057963 | ENSMUST00000046518 |
| 13 | Sh3rf3 | SH3 domain containing ring finger 3 | ENSMUSG00000037990 | ENSMUST00000153031 |
| 14 | Mical3 | microtubule associated monooxygenase, calponin and LIM domain containing 3 | ENSMUSG00000051586 | ENSMUST00000238968 |
| 15 | Abcc8 | ATP-binding cassette, sub-family C member 8 | ENSMUSG00000040136 | ENSMUST00000033123 |
| 16 | Flnb | filamin, beta | ENSMUSG00000025278 | ENSMUST00000052678 |
| 17 | Lime1 | Lck interacting transmembrane adaptor 1 | ENSMUSG00000090077 | ENSMUST00000048077 |
| 18 | Zswim4 | zinc finger SWIM-type containing 4 | ENSMUSG00000035671 | ENSMUST00000039480 |
| 19 | Cdc42bpg | CDC42 binding protein kinase gamma | ENSMUSG00000024769 | ENSMUST00000025681 |
| 20 | Kcnh1 | potassium voltage-gated channel, subfamily H (eag-related), member 1 | ENSMUSG00000058248 | ENSMUST00000078470 |
| 21 | Col5a1 | collagen, type V, alpha 1 | ENSMUSG00000026837 | ENSMUST00000028280 |
| 22 | Adamts17 | ADAM metallopeptidase with thrombospondin type 1 motif 17 | ENSMUSG00000058145 | ENSMUST00000098382 |
| 23 | Smoc2 | SPARC related modular calcium binding 2 | ENSMUSG00000023886 | ENSMUST00000233344 |
| 24 | Colla1 | collagen, type I, alpha 1 | ENSMUSG00000001506 | ENSMUST00000001547 |
| 25 | Wnt6 | wingless-type MMTV integration site family, member 6 | ENSMUSG00000033227 | ENSMUST00000006716 |
| 26 | Col1a2 | collagen, type I, alpha 2 | ENSMUSG00000029661 | ENSMUST00000031668 |
| 27 | Ccbe1 | collagen and calcium binding EGF domains 1 | ENSMUSG00000046318 | ENSMUST00000130300 |
| 28 | Tgfbr3 | transforming growth factor, beta receptor III | ENSMUSG00000029287 | ENSMUST00000031224 |
| 29 | Mgp | matrix Gla protein | ENSMUSG00000030218 | ENSMUST00000032342 |
| 30 | Dmp1 | dentin matrix protein 1 | ENSMUSG00000029307 | ENSMUST00000066708 |
| 31 | Tnxb | tenascin XB | ENSMUSG00000033327 | ENSMUST00000238967 |
| 32 | Pxdn | peroxidasin | ENSMUSG00000020674 | ENSMUST00000122328 |
| 33 | Lamb1 | laminin B1 | ENSMUSG00000002900 | ENSMUST00000239496 |
| 34 | Omd | osteomodulin | ENSMUSG00000048368 | ENSMUST00000221170 |
| 35 | Rtn4rl2 | reticulon 4 receptor-like 2 | ENSMUSG00000050896 | ENSMUST00000151799 |
| 36 | Ltbp4 | latent transforming growth factor beta binding protein 4 | ENSMUSG00000040488 | ENSMUST00000038618 |
| 37 | Tgfb2 | transforming growth factor, beta 2 | ENSMUSG00000039239 | ENSMUST00000195201 |
| 38 | Fbln1 | fibulin 1 | ENSMUSG00000006369 | ENSMUST00000057410 |

[2] The prophylactic and/or therapeutic agent for neurodegenerative diseases according to [1], wherein the inhibitor is;
(A) a vector containing a polynucleotide comprising a nucleic acid sequence of a miR-shRNA (microRNA adapted short hairpin RNA) against any of the genes listed in Table 1, and a promoter sequence operably linked to the nucleic acid sequence,
(B) siRNA, shRNA, or antisense oligonucleotides against any of the genes listed in Table 1,
(C) an antibody against a protein derived from any of the genes listed in Table 1, or
(D) a small molecule which has an inhibitory effect on the expression and/or function of any of the genes listed in Table 1 or of proteins derived therefrom.

[3] The prophylactic and/or therapeutic agent for neurodegenerative diseases according to [1] or [2], wherein the genes are mammalian genes.
[4] The prophylactic and/or therapeutic agent for neurodegenerative diseases according to [2] or [3], wherein the vector is lentiviral vector, adeno-associated viral vector, adenoviral vector, or plasmid vector.
[5] The prophylactic and/or therapeutic agent for neurodegenerative diseases according to any of [1] to [4], which is administered by lumbar puncture.
[6] The prophylactic and/or therapeutic agent for neurodegenerative diseases, comprising as an active ingredient a promoter of the expression and/or function of any of the ligand-receptor gene sets listed in Table 2 below or of a protein derived from any of the gene sets.

**[Table 2]**

| ID | Ligand/ Receptor | Official Symbol | Gene Name | Ensembl Gene ID | Transcript ID |
|---|---|---|---|---|---|
| 1 | Ligand | Apoe | apolipoprotein E | ENSMUSG00000002985 | ENSMUST00000174064 |
| | Receptor | Abca1 | ATP-binding cassette, sub-family A member 1 | ENSMUSG00000015243 | ENSMUST00000030010 |
| 2 | Ligand | Il33 | interleukin 33 | ENSMUSG00000024810 | ENSMUST00000025724 |
| | Receptor | Il1rl1 | interleukin 1 receptor-like 1 | ENSMUSG00000026069 | ENSMUST00000097772 |
| 3 | Ligand | Adam17 | a disintegrin and metallopeptidase domain 17 | ENSMUSG00000052593 | ENSMUST00000101551 |
| | Receptor | Notch2 | notch 2 | ENSMUSG00000027878 | ENSMUST00000079812 |
| 4 | Ligand | Ccl3 | C-C motif chemokine ligand 3 | ENSMUSG00000000982 | ENSMUST00000001008 |
| | Receptor | Ccr5 | C-C motif chemokine receptor 5 | ENSMUSG00000079227 | ENSMUST00000111442 |
| 5 | Ligand | Apoe | apolipoprotein E | ENSMUSG00000002985 | ENSMUST00000174064 |
| | Receptor | Trem2 | triggering receptor expressed on myeloid cells 2 | ENSMUSG00000023992 | ENSMUST00000113237 |
| 6 | Ligand | C3 | complement component 3 | ENSMUSG00000024164 | ENSMUST00000024988 |
| | Receptor | Itgb2 | integrin beta 2 | ENSMUSG00000000290 | ENSMUST00000000299 |
| 7 | Ligand | Icam1 | intercellular adhesion molecule 1 | ENSMUSG00000037405 | ENSMUST00000086399 |
| | Receptor | Itgb2 | integrin beta 2 | ENSMUSG00000000290 | ENSMUST00000000299 |
| 8 | Ligand | Adam17 | a disintegrin and metallopeptidase domain 17 | ENSMUSG00000052593 | ENSMUST00000101551 |
| | Receptor | Tnfrsf1a | tumor necrosis factor receptor superfamily, member 1a | ENSMUSG00000030341 | ENSMUST00000032491 |
| 9 | Ligand | Pros1 | protein S (alpha) | ENSMUSG00000022912 | ENSMUST00000023629 |
| | Receptor | Mertk | MER proto-oncogene tyrosine kinase | ENSMUSG00000014361 | ENSMUST00000014505 |
| 10 | Ligand | Lgals3 | lectin, galactose binding, soluble 3 | ENSMUSG00000050335 | ENSMUST00000142734 |
| | Receptor | Mertk | MER proto-oncogene tyrosine kinase | ENSMUSG00000014361 | ENSMUST00000014505 |

[7] The prophylactic and/or therapeutic agent for neurodegenerative diseases according to any of [1] to [6], wherein the neurodegenerative disease is at least one selected from the group consisting of Alzheimer's disease, Parkinson's disease, Huntington's disease, and dementias caused by frontotemporal lobar degeneration.
[8] The prophylactic and/or therapeutic agent for neurodegenerative diseases according to any of [1] to [6], wherein the neurodegenerative disease is at least one selected from the group consisting of Alzheimer's disease and Parkinson's disease.
[9] The prophylactic and/or therapeutic agent for neurodegenerative diseases according to any of [1] to [6], wherein the neurodegenerative disease is Alzheimer's disease.

### Advantageous Effects of Invention

According to the present invention, it is possible to efficiently activate neurogenesis in the adult brain and reduce the deposition of amyloid-*β* by suppressing or inhibiting the expression of the specific genes above or of the proteins derived from any of these genes. Since the activation of neurogenesis and the reduction of amyloid-*β* deposition in the adult brain are thought to lead to improvements in age-related cognitive impairments and neurodegenerative diseases, the knockdown of the specific genes above such as Prkag2, Gfra2, Maml2, Rp129, or proteins derived from any of these genes is strongly suggested to be a promising therapeutic strategy for neurodegenerative diseases, including Alzheimer's disease.

### BRIEF DESCRIPTION OF DRAWINGS

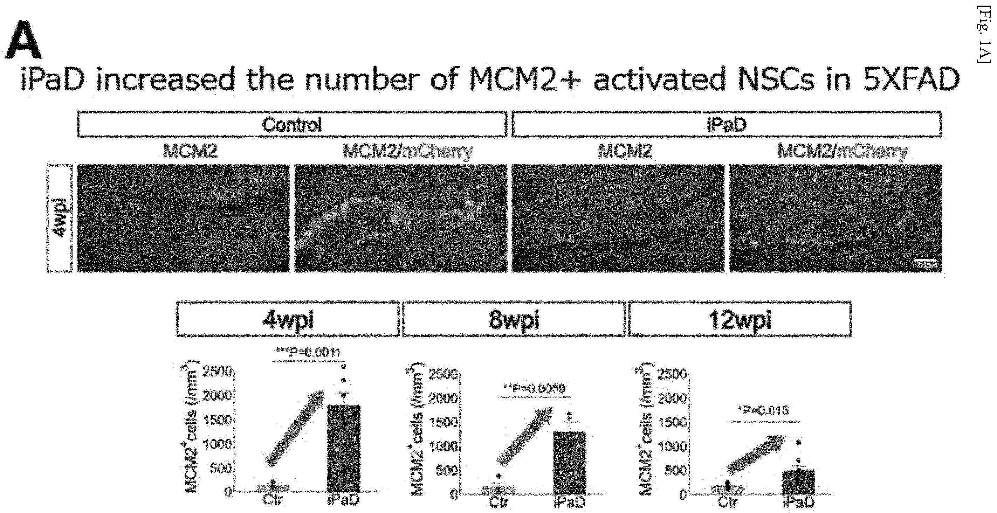

[Fig. 1A] Fig. 1A shows a long-term activation of neurogenesis in the brains of Alzheimer's disease model mice by iPaD treatment. Control or iPaD lentivirus was injected into the hippocampal dentate gyrus of 5-month-old 5XFAD mice, and brain sections were immunohistochemically examined at subsequent stages (4, 8, and 12 weeks post-infection (wpi)). Quantitative data of MCM2+ cells are shown in the lower panel, and MCM2 expression at 4 wpi is shown in the upper panel. NSCs infected with the lentivirus and their progeny cells are mCherry+. Due to iPaD treatment, the number of MCM2+ activated NSCs increased compared to the control. N = 4.
[Fig. 1B] Fig. 1B shows, from the same experiment as Fig. 1A, quantitative data of DCX+ cells in the lower panel and DCX expression at 4 wpi in the upper panel. NSCs infected with the lentivirus and their progeny cells are mCherry+. Due to iPaD treatment, the number of DCX+ newborn neuroblasts increased compared to the control. N = 4.
[Fig. 2] Fig. 2 shows a suppressive effect of iPaD treatment on A*β* deposition in the brains of Alzheimer's disease model mice. Control or iPaD lentivirus was injected into the hippocampal dentate gyrus of 5-month-old 5XFAD mice, and brain sections were immunohistochemically examined at subsequent stages (4, 8, and 12 wpi). Quantitative data of A*β* deposition is shown on the right. iPaD treatment reduced A*β* deposition. N = 4.
[Fig. 3] Fig. 3 shows the genes whose expressions increased or decreased in the mouse hippocampus due to iPaD treatment. Control or iPaD lentivirus was injected into the hippocampal dentate gyrus of 5-month-old 5XFAD mice, and 4 weeks later (4 wpi), the hippocampus was isolated and subjected to RNA-seq analysis. In the hippocampus of 5XFAD mice, many genes showed significant increase or decrease of expression due to iPaD treatment (p-value < 0.05).
[Fig. 4A] Fig. 4A shows the results of gene ontology (GO) analysis of 592 genes whose expression significantly increased due to iPaD treatment in the hippocampus of 5XFAD mice.
[Fig. 4B] Fig. 4B shows the results of gene ontology (GO) analysis of 462 genes whose expression significantly decreased due to iPaD treatment in the hippocampus of 5XFAD mice.
[Fig. 5] Fig. 5 shows a knockdown strategy using miR-E-based shRNA system, in which mCherry and a nuclear localization signal (3NLS) are repeated three times under the control of ubiquitous elongation factor promoter (pEFS). This knockdown system was introduced into the hippocampal dentate gyrus of 5XFAD mice using lentivirus.
[Fig. 6A] Fig. 6A shows the comparison of neurogenesis by the knockdown of Maml2, Ptgds, Ell3, Prkag2, Rp129, Gfra2, Itpk1, and Mical3 in 5XFAD mice. It shows the results of examining the brains 8 weeks after administering lentiviruses for the knockdown of each gene or a control to 5 to 6-month-old 5XFAD mice. Knockdown of Prkag2 most effectively activated neurogenesis. N = 4.
[Fig. 6B] Fig. 6B shows, from the same experiment as Fig. 6A, quantitative data of DCX+ cells. Knockdown of Prkag2 most effectively activated neurogenesis. N = 4.
[Fig. 7A] Fig. 7A shows the effect of reducing A*β* deposition due to the knockdown of Maml2, Ptgds, Ell3, Prkag2, Rp129, Gfra2, Itpk1, and Mical3 in 5XFAD mice. It shows the results of examining the brains 8 weeks after administering lentiviruses for the knockdown of each gene or a control to 5 to 6-month-old 5XFAD mice. Knockdown of Prkag2 and Gfra2 most effectively reduced A*β* deposition. N = 4.
[Fig. 7B] Fig. 7B shows, from the same experiment as Fig. 7A, quantitative data of A*β* deposition. Knockdown of Prkag2 and Gfra2 most effectively reduced A*β* deposition. N = 4.
[Fig. 8] Fig. 8 shows tissue staining images of lipid droplets (white dots of BODIPY) and activated AMPK (white dots of pAMPK) in 4-month-old mice (wild type and 5XFAD) and 8-month-old mice (5XFAD).
[Fig. 9] Fig. 9 shows the results of measuring by Western blot the amount of pAMPK in cell extracts two days after infecting cultured neural stem cells with Prkag2 knockdown lentivirus (Prkag2 shRNA) or control lentivirus (Control) and collecting the cell extracts.
[Fig. 10] Fig. 10 shows the results of measuring by qPCR the amount of mRNA of each AMPK subunit in cell extracts obtained in the same manner as in Fig. 9.
[Fig. 11] Fig. 11 shows the results of quantifying EdU-incorporating cells two days after infecting cultured neural stem cells with Prkag2 knockdown lentivirus (Prkag2 shRNA), Maml2 knockdown lentivirus (Maml2 shRNA), or control lentivirus (Control).
[Fig. 12] Fig. 12 schematically shows the results of a search for secretory factor-receptor pairs involved in AMPK signaling among the gene groups activated by iPaD. As a result, the pairs Adipoq-Adipor2, Il33-Il1rl1, and Il17a-Il17rc were identified.
[Fig. 13] Fig. 13 shows the results of collecting cell extracts two days after infecting cultured neural stem cells with iPaD lentivirus (iPaD) or control lentivirus (Control), and measuring by qPCR the amount of mRNA of secretory factors involved in the AMPK signaling.

### DESCRIPTION OF EMBODIMENTS

Hereinafter, the present invention is described in detail. As used herein, molecular biological techniques can be carried out by methods described in generally known experimental manuals for those skilled in the art, or by methods equivalent thereto, unless otherwise specifically stated. Also, as used herein, the terms shall be construed as having the meanings generally used in the relevant technical field, unless otherwise specified.

The present inventors previously demonstrated that administration of lentivirus with Plagl2 expression-inducing activity and anti-Dyrk1a activity (iPaD) to 5XFAD mice, model mice of Alzheimer's disease, efficiently activates neurogenesis in the adult brain, reduces amyloid-*β* deposition, and improves cognitive function. Based on these research results, they have further advanced their research and investigated the downstream events of iPaD treatment in 5XFAD mice. Through global RNA-seq and functional analyses, it has been revealed that iPaD treatment in 5XFAD mice significantly reduces the expression of specific genes listed in Table 1 and significantly enhances the expression of specific genes (ligand-receptor pairs) listed in Table 2. When Prkag2 (protein kinase AMP-activated non-catalytic subunit gamma 2), Maml2 (mastermind like transcriptional coactivator 2), Gfra2 (glial cell line-derived neurotrophic factor receptor alpha-2), Rpl29 (ribosomal protein L29), among the specific genes above whose expressions were reduced by iPaD treatment, were each knocked down, the obtained results have shown that neurogenesis in the adult brain was efficiently activated and the deposition of amyloid-*β* was reduced. As the activation of neurogenesis in the adult brain has a potential to improve age-related cognitive impairments and neurodegenerative diseases, it has been strongly suggested that inhibitors of specific genes such as Prkag2 and Maml2 listed in Table 1 above or of proteins derived from any of these genes, as well as agents which promote the expression and/or function of specific ligand-receptor gene sets listed in Table 2 above or of proteins derived from any of these gene sets are promising therapeutic strategies for such neurodegenerative diseases, including Alzheimer's disease.

### <Prophylactic and/or therapeutic agent for neurodegenerative diseases>

The first embodiment of the prophylactic and/or therapeutic agent for neurodegenerative diseases of the present invention is characterized in that it comprises, as an active ingredient, an inhibitor of any of the genes listed in Table 1 above or of a protein derived from any of these genes. Furthermore, the second embodiment of the prophylactic and/or therapeutic agent for neurodegenerative diseases of the present invention is characterized in that it comprises, as an active ingredient, a promoter of the expression and/or function of any of the ligand-receptor gene sets listed in Table 2 above or of proteins derived from any of these gene sets. The following explains each of the embodiments.

### [The first embodiment]

The first embodiment of the prophylactic and/or therapeutic agent for neurodegenerative diseases of the present invention is characterized in that it comprises, as an active ingredient, an inhibitor of any of the genes listed in Table 1 above or of a protein derived from any of these genes.

As the nucleic acid sequences of the genes in Table 1 above, the known sequences of the coding regions (CDS) of the mRNA sequences of each of the genes above can be adopted, and for example, the transcript sequences of each gene registered in the gene database Ensembl can be used. The nucleic acid sequences of the genes in Table 1 in the present invention are identical to, or have 80% or more, preferably 90% or more, more preferably 95% or more, further preferably 98% or more, particularly preferably 99% or more homology to the transcript sequences of the corresponding genes registered in Ensembl, and the proteins encoded by these genes have the activity corresponding to each gene registered in nucleotide sequence databases of Ensembl and the like.

The genes listed in Table 1 above are derived from mammals such as humans, monkeys, pigs, horses, cows, rabbits, sheep, goats, cats, dogs, guinea pigs, or mice; preferably from humans, monkeys, or mice; and more preferably from humans.

The inhibitors of the present invention are not particularly limited as long as they inhibit or suppress, in whole or in part, the expression and/or function of any of the genes listed in Table 1 or proteins derived therefrom; preferred examples include the following.
(A) A vector containing a polynucleotide comprising a nucleic acid sequence of a miR-shRNA (microRNA adapted short hairpin RNA) against any of the genes listed in Table 1 above, and a promoter sequence operably linked to the above nucleic acid sequence.
(B) siRNA, shRNA, or antisense oligonucleotides against any of the genes listed in Table 1 above.
(C) An antibody against a protein derived from any of the genes listed in Table 1 above.
(D) A small molecule which has an inhibitory effect on the expression and/or function of any of the genes listed in Table 1 above or of proteins derived therefrom.

The following explains each of them.

### (A) A vector containing a polynucleotide comprising a nucleic acid sequence of a miR-shRNA (microRNA adapted short hairpin RNA) against any of the genes listed in Table 1 above, and a promoter sequence operably linked to the nucleic acid sequence above.

miR-shRNA (microRNA adapted short hairpin RNA) against any of the genes listed in Table 1 above is one of the shRNAs (hairpin-shaped RNA sequences used for gene silencing via RNA interference) which can suppress the expression of the genes above. shRNA is RNA which can be processed by Dicer in the body to produce siRNA. shRNA has a stem-loop structure which comprises a double-stranded stem and a hairpin loop. The sequence of this hairpin loop portion is not particularly limited, but it can be a sequence of 5 to 12 bases (Kawasaki H. et al., Nucleic Acid Res. (2003) 31: 700-707). miR-shRNA is an shRNA adapted to the miRNA pathway, expressed by RNA polymerase II, and exhibits its effect by being cleaved by both Drosha and Dicer. Such miR-shRNA is not particularly limited as long as it has a nucleic acid sequence capable of suppressing the expression of the genes above, and it can be designed based on the gene sequences listed in Table 1 above using methods well known to those skilled in the art, and can be appropriately manufactured.

In this embodiment, a promoter sequence is operably linked to the nucleic acid sequence of the miR-shRNA above. Here, as the promoter sequence in the "operably linked promoter sequence," a promoter sequence which functions in neural system cells, such as neural stem cells, can be adopted. Such promoter sequences include, for example, Hes5 promoter, GFAP promoter, Sox2 promoter, Lfng promoter, U6 promoter, H1 promoter, etc., which function in neural stem cells.

Here, "operably linked" refers to an arrangement in which the described ingredients are in a relationship which enables them to function in their intended manner. In one embodiment, this term refers to the functional linkage between a nucleic acid expression control sequence (for example, a promoter and/or an enhancer) and a desired polynucleotide sequence, and which functional linkage can be either a direct linkage or an indirect linkage (where another polynucleotide sequence intervenes therebetween). In the present disclosure, the promoter sequence directs the transcription of the nucleic acid sequence of the linked target miR-shRNA (microRNA adapted short hairpin RNA).

As used herein, the term "polynucleotide" is used interchangeably with "nucleic acid," "gene," or "nucleic acid molecule," and refers to a polymer of nucleotides. As used herein, the term "nucleotide sequence" is used interchangeably with "nucleic acid sequence" or "base sequence" and is shown as a sequence of deoxyribonucleotides (abbreviated as A, G, C, and T). For example, when stating "a polynucleotide or a fragment thereof comprising the nucleotide sequence of SEQ ID NO: 1," it refers to a polynucleotide or a fragment portion thereof comprising the sequence represented by each deoxynucleotide A, G, C, and/or T of SEQ ID NO: 1.

In the present invention, the polynucleotide can further comprise known sequences such as enhancer sequences which assist in transcription of mRNA and translation into protein; Kozak sequences; and appropriate polyadenylation signal sequences.

In the present invention, the means for introducing a polynucleotide into target cells such as neural stem cells is not particularly limited; examples include methods using vectors such as viruses, plasmids, or artificial chromosomes, and techniques such as lipofection, liposome-mediated transfection, and microinjection.

When introducing the polynucleotide of the present embodiment into target cells, it is preferable to use a vector among the above. The vectors used in the present invention are not particularly limited as far as they are capable of efficiently delivering genes into the target cells (such as neural stem cells). Examples of such vectors include viral vectors such as lentiviral vectors, adeno-associated viral vectors, adenoviral vectors, herpesvirus vectors (for example, herpes simplex virus vectors), poxvirus vectors, baculovirus vectors, papillomavirus vectors, and papovavirus vectors (for example, SV40); plasmid vectors; phagemid vectors; cosmid vectors; and bacteriophages such as lambda phage and M13 phage. Among these, lentiviral vectors, adeno-associated viral vectors, adenoviral vectors, and plasmid vectors are preferred, lentiviral vectors and adeno-associated viral vectors are more preferred, and lentiviral vectors are further preferred.

Specific examples of expression vectors include pLenti4/V5-DEST(TM), pLenti6/V5-DEST(TM), and pLenti6.2/V5-GW/lacZ (Invitrogen) for lentivirus-mediated gene introduction and expression in mammalian cells; as well as the pClneo vector (Promega) for expression in mammalian cells.

The vector can comprise, in addition to the above-mentioned promoters and enhancers, control sequences such as ribosome binding sequences, terminators, and polyadenylation sites, and the like, so that the target gene can be expressed. Also, the vector can comprise, as needed, selection marker sequences such as drug resistance genes (for example, kanamycin resistance gene, ampicillin resistance gene, puromycin resistance gene, etc.), thymidine kinase gene, diphtheria toxin gene; reporter gene sequences such as mCherry (red fluorescent protein), green fluorescent protein (GFP), *β*-glucuronidase (GUS), FLAG; and the like.

When the vector comprising the miR-shRNA, the promoter above, and the like of this embodiment is introduced into target neural stem cells, neurons, astrocytes, oligodendrocytes, etc., to transform them, the genes listed in Table 1 above can be knocked down. Target cells such as neural stem cells which have been transformed in this way can become activated, proliferate, and differentiate in a manner that allows them to fully demonstrate their cellular functions, such as generating a large number of neurons. The above vector of the present embodiment can be suitably used for neural stem cells and the like inherent in adult brains, aged brains, diseased brains, and the like.

### (B) siRNA, shRNA, or antisense oligonucleotides against any of the genes listed in Table 1 above

The inhibitors contained in the prophylactic and/or therapeutic agent for neurodegenerative diseases of the present invention include siRNA (small interfering RNA), shRNA (small hairpin RNA), antisense oligonucleotides, dsRNA (double-stranded RNA), miRNA (microRNA), and the like against any of the genes described in Table 1 above. From the perspective of the effects of the present invention, among these, siRNA, shRNA, and antisense oligonucleotides are preferred.
siRNA is a double-stranded oligoribonucleotide consisting of an RNA having a sequence complementary to the nucleotide sequence or a part of the nucleotide sequence (target nucleotide sequence) of the mRNA of a target gene and a complementary strand thereof. In addition, shRNA, which is a single-stranded RNA in which a sequence complementary to a target nucleotide sequence (first sequence) and a sequence complementary thereto (second sequence) are linked via a hairpin loop portion so that the first sequence forms a double-stranded structure with the second sequence, and a dumbbell-shaped nucleic acid in which both ends of the double-stranded structure formed by the first and second sequences are closed by loop structures, are also included in siRNA.
siRNA can have overhangs at one or both of the 5' or 3' ends of the sense strand and antisense strand. An overhang is formed by the addition of one or several bases (for example, 1, 2, or 3 bases) at the ends of the sense strand and/or antisense strand. The base length of siRNA is not particularly limited as long as it can induce RNA interference, and is, for example, 10 to 50 bases, 15 to 30 bases, or 21 to 27 bases on one strand.
siRNA is not particularly limited as long as it targets the mRNA corresponding to the genes listed in Table 1 above; it can be designed based on the base sequence information of the mRNA; and it can induce RNA interference. siRNA can be chemically synthesized using known techniques or produced using genetic engineering techniques.
shRNA is RNA which can be processed by Dicer in vivo to produce siRNA. shRNA has a stem-loop structure comprising a double-stranded stem and a hairpin loop. The sequence of this hairpin loop part is not particularly limited, but it can be a sequence of 5 to 12 bases (Kawasaki H. et al., Nucleic Acids Res. (2003) 31: 700-707). The shRNA described here refers to shRNA other than the miR-shRNA in (A) mentioned above and is classified as Simple-stem-loop-based shRNA. This shRNA is expressed by RNA polymerase III, cleaved by Dicer, and exhibits its effect. Such shRNA can be designed and manufactured based on the gene sequences shown in Table 1 above by methods well known to those skilled in the art.

The sequence of antisense oligonucleotides (antisense nucleic acids) is preferably complementary to the target gene or a part thereof. However, as long as the expression of the gene can be effectively suppressed, it does not need to be perfectly complementary; it is sufficient if it has, preferably 90% or more complementarity; more preferably 95% or more complementarity to the transcript of the target gene. To effectively suppress the expression of the target gene using antisense nucleic acids, the length of the antisense nucleic acid is preferably 15 bases or more. Furthermore, to avoid non-specific influences, it is preferable to use multiple antisense nucleic acids complementary to different sequences of the target gene. Antisense nucleic acids in the present invention include not only nucleic acids comprising antisense sequences of the translated region of the target gene but also nucleic acids comprising antisense sequences of untranslated regions of the target gene.
dsRNA has an RNAi effect and is composed of sense RNA which has a sequence identical to any continuous RNA region of the mRNA corresponding to the gene listed in Table 1 above, and antisense RNA which has a sequence complementary to the sense RNA. The length of the "any continuous RNA region" is usually 20 to 30 bases, and preferably 21 to 23 bases.
miRNA refers to a non-coding RNA with the length of 15 to 25 bases, which is transcribed as a hairpin-like RNA precursor, cleaved by a dsRNA-cleaving enzyme with RNase III cleavage activity, incorporated into a protein complex called RISC, and involved in the suppression of mRNA translation. The miRNA in the present invention also includes precursors of the miRNA (pre-miRNA, pri-miRNA).

For these siRNAs (small interfering RNAs), shRNAs (small hairpin RNAs), antisense oligonucleotides, dsRNAs (double-stranded RNAs), miRNAs (microRNAs), and the like, it is also possible to use expression vectors which expresses them. In the case of using expression vectors, the vectors can comprise regulatory sequences such as promoters, enhancers, terminators, and the like so that the target genes above can be expressed. Additionally, if necessary, the vectors can comprise selection marker sequences such as drug resistance genes (for example, kanamycin resistance gene, ampicillin resistance gene, puromycin resistance gene, etc.), thymidine kinase gene, diphtheria toxin gene; reporter gene sequences such as mCherry (red fluorescent protein), green fluorescent protein (GFP), *β-*glucuronidase (GUS), FLAG; and the like.

### (C) An antibody against a protein derived from any of the genes listed in Table 1 above.

The inhibitors contained in the prophylactic and/or therapeutic agent for neurodegenerative diseases of the present invention can include an antibody against a protein derived from any of the genes listed in Table 1. The protein derived from the gene above refers to the protein which has the amino acid sequence encoded by the gene above. As the inhibitor above, an antibody against the protein derived from the gene above or against the protein having the amino acid sequence encoded by the gene above, which inhibits, suppresses, or reduces the function of this protein, is preferable.

In the present invention, an "antibody" refers to a protein comprising one or more polypeptides which are substantially or partially encoded by an immunoglobulin gene or a fragment of an immunoglobulin gene, and comprises one or more molecules capable of binding to a specific epitope on an antigen. In the present invention, the form of the "antibody" can be a full-length immunoglobulin (an antibody having an Fc region and Fab region, a full-length antibody), or can be F(ab')2, Fab', Fab, Fv antibodies (variable fragment of antibody), disulfide-bonded Fv (dsFv), single-chain antibodies (scFv), or polymers thereof (for example, diabodies). The antibody can be a monoclonal antibody or a polyclonal antibody. The antibody can, for example, be a chimeric antibody, a humanized antibody, or a fully humanized antibody. The antibody can, for example, be a bispecific or oligospecific antibody. These antibodies can be manufactured by methods conventionally known to those skilled in the art.

### (D) A small molecule which has an inhibitory effect on the expression and/or function of any of the genes listed in Table 1 above or of proteins derived therefrom.

The inhibitors contained in the prophylactic and/or therapeutic agent for neurodegenerative diseases of the present invention include a small molecule which has an inhibitory effect on the expression and/or function of any of the genes listed in Table 1 above or of the proteins derived therefrom.

The small molecule above can be found from a compound library or the like through methods for screening such as the following.

A method for screening comprising
(1) the step of exposing neural stem cells to a test substance and measuring the expression of any of the genes listed in Table 1 above or of the proteins derived from the above genes; and
(2) the step of selecting a test substance which exhibits an expression suppressive effect on the genes above based on the results of step (1) above.

The substances selected by the screening method above can efficiently activate neural stem cells, promote their proliferation, and produce a large number of neurons. Therefore, it is possible to restore neural stem cells in the aged brain to a state seen in adolescence or an even younger period, produce a large number of neurons, and potentially improve memory and learning abilities. In addition, therapeutic effects on neurodegenerative diseases such as Alzheimer's disease are also expected, as the endogenous neural stem cells can be efficiently activated to produce a large number of neurons, thereby improving memory and learning abilities.

### [The second embodiment]

The second embodiment of the prophylactic and/or therapeutic agent for neurodegenerative diseases of the present invention is characterized in that it comprises, as an active ingredient, a promoter of the expression and/or function of any of the ligand-receptor gene sets listed in Table 2 above or of proteins derived from any of these gene sets. The sets of ligands and receptors listed in Table 2 above are activated in the downstream of iPaD treatment, as shown in the examples described below. Specifically, when dormant neural stem cells are activated by iPaD treatment, it is expected that the increase in secretion and activation of the corresponding ligands in the ligand-receptor set above in the hippocampus are caused, and that the ligands act on surrounding cells to suppress the deposition of amyloid *β* (Aβ). The prophylactic and/or therapeutic agent for neurodegenerative diseases of the present invention, similarly to iPaD treatment, comprises as an active ingredient a substance which exhibits an effect of promoting (activating) the expression and/or function of the ligand-receptor sets above or of any of the ligands or receptors above.

As the nucleic acid sequences of the genes in Table 2 above, the known sequences of the coding regions (CDS) of the mRNA sequences of each of the above genes can be adopted, and for example, the transcript sequences of each gene registered in the nucleotide sequence database Ensembl can be used. The nucleic acid sequences of the genes in Table 2 in the present invention are identical to, or have 80% or more, preferably 90% or more, more preferably 95% or more, further preferably 98% or more, particularly preferably 99% or more homology to the transcript sequences of the corresponding genes registered in Ensembl, and the peptides encoded by these have the activity corresponding to each gene registered in nucleotide sequence databases of Ensembl.

The genes listed in Table 2 above are derived from mammals such as humans, monkeys, pigs, horses, cows, rabbits, sheep, goats, cats, dogs, guinea pigs, or mice; preferably from humans, monkeys, or mice; and more preferably from humans.

The promoters above are not particularly limited as long as they promote the expression and/or function of any of the genes listed in Table 2 above or of the proteins derived from any of these genes.

### [Usage, dosage specific target disease, etc. of the prophylactic and/or therapeutic agent for neurodegenerative diseases]

The prophylactic and/or therapeutic agent for neurodegenerative diseases of the present invention is administered to the subject by lumbar puncture, intracerebroventricular injection, bolus injection or infusion into the cerebrospinal cavity, intraganglionic injection, intraneural injection, or subcutaneous injection.

The dosage of the prophylactic and/or therapeutic agent for neurodegenerative diseases of the present invention is not particularly limited, and it is possible to select an appropriate dosage according to various conditions such as the type of disease, the age and symptoms of the patient, the route of administration, the purpose of treatment, and the presence or absence of concomitant drugs.

Specific diseases for which the prophylactic and/or therapeutic agent for neurodegenerative diseases of the present invention is effective include Alzheimer's disease, Parkinson's disease, Huntington's disease, dementias caused by frontotemporal lobar degeneration, and the like. Among these, from the perspective that the effect of the prophylactic and/or therapeutic agent for neurodegenerative diseases of the present invention can be more effectively obtained, it is suitably used for Alzheimer's disease and Parkinson's disease.

### <Therapeutic Method >

The present invention also includes a prophylactic and/or therapeutic method for neurodegenerative diseases, characterized by using an inhibitor of any of the genes listed in Table 1 above or of a protein derived from any of these genes, for the prophylaxis and/or therapy for neurodegenerative diseases. Furthermore, the present invention also includes a prophylactic and/or therapeutic method for neurodegenerative diseases, characterized by using a promoter of the expression and/or function of any of the ligand-receptor gene sets listed in Table 2 above or of proteins derived from any of these gene sets for the prophylaxis and/or therapy for neurodegenerative diseases.

The prophylactic and/or therapeutic method for neurodegenerative diseases of the present invention is as follows.
[1] A prophylactic and/or therapeutic method for neurodegenerative diseases, characterized by administering an inhibitor of any of the genes listed in Table 1 above or of a protein derived from any of these genes.
[2] The prophylactic and/or therapeutic method for neurodegenerative diseases according to [1], wherein the inhibitor above is;
   (A) a vector containing a polynucleotide comprising a nucleic acid sequence of a miR-shRNA (microRNA adapted short hairpin RNA) against any of the genes listed in Table 1 above, and a promoter sequence operably linked to the nucleic acid sequence above,
   (B) siRNA, shRNA, or antisense oligonucleotides against any of the genes listed in Table 1 above,
   (C) an antibody against a protein derived from any of the genes listed in Table 1 above, or
   (D) a small molecule which has an inhibitory effect on the expression and/or function of any of the genes listed in Table 1 above or of proteins derived therefrom.
[3] The prophylactic and/or therapeutic method for neurodegenerative diseases according to [1] or [2], wherein the genes above are mammalian genes.
[4] The prophylactic and/or therapeutic method for neurodegenerative diseases according to [2] or [3], wherein the vector above is lentiviral vector, adeno-associated viral vector, adenoviral vector, or plasmid vector.
[5] The prophylactic and/or therapeutic method for neurodegenerative diseases according to any of [1] to [4], which is administered by lumbar puncture.
[6] The prophylactic and/or therapeutic method for neurodegenerative diseases, characterized by administering a promoter of the expression and/or function of a protein derived from any of the ligand-receptor gene sets listed in Table 2 above or from any of these gene sets.
[7] The prophylactic and/or therapeutic method for neurodegenerative diseases according to any of[1] to [6], wherein the neurodegenerative disease above is at least one selected from the group consisting of Alzheimer's disease, Parkinson's disease, Huntington's disease, and dementias caused by frontotemporal lobar degeneration.
[8] The prophylactic and/or therapeutic method for neurodegenerative diseases according to any of [1] to [6], wherein the neurodegenerative disease above is at least one selected from the group consisting of Alzheimer's disease and Parkinson's disease.
[9] The prophylactic and/or therapeutic method for neurodegenerative diseases according to any of [1] to [6], wherein the neurodegenerative disease above is Alzheimer's disease.

The aforementioned-description of the prophylactic and/or therapeutic agent for neurodegenerative diseases can directly apply to the description in detail of the prophylactic and/or therapeutic agent for neurodegenerative diseases of the present invention.

### <Method for screening prophylactic and/or therapeutic agents for neurodegenerative diseases>

The first embodiment of the present invention comprises a method for screening prophylactic and/or therapeutic agents for neurodegenerative diseases, comprising:
(1) the step of exposing neural stem cells to a test substance and measuring the expression of any of the genes listed in Table 1 above or the expression and/or activity of the proteins derived from the above genes; and
(2) the step of selecting a test substance which exhibits an expression suppressive effect on the genes above or; an expression suppressive effect or activity suppressive effect on a protein derived from the above genes, based on the results of step (1) above.

As the neural stem cells above, in addition to primary cultured cells of neural stem cells derived from various animals, neural stem cells differentiated from stem cells such as ES cells or iPS cells using well known methods, or cell lines such as the mouse neural stem cell line including MEB5 can also be used.

The test substances in the present invention are not particularly limited and can include, for example, single substances such as natural compounds, organic compounds, inorganic compounds, nucleic acids, proteins (including antibodies), and peptides; expression products of compound libraries, nucleic acid libraries, peptide libraries, or gene libraries; extracts such as cell extracts, cell culture supernatants, fermentation products of microorganisms, marine organism extracts, plant extracts, prokaryotic cell extracts, eukaryotic unicellular extracts, or animal cell extracts. Also, the test substances in the present invention can be mixtures of these above.

As a method for measuring the expression of any of the genes listed in Table 1 above or the expression and/or activity of the proteins derived from the genes above, any conventionally known method for measuring gene expression, protein expression, or protein activity in various cells can be used. The method can include, for example, measuring the expression of each gene, the expression and/or activity of each protein in the presence and absence of the test substance, and assessing the measured result based on differences between the presence and absence of the test substance or on the ratio of the two, and the like. Using these methods and the like, test substances which exhibit an expression suppressive effect on the genes above or; an expression suppressive effect or activity suppressive effect on a protein derived from the genes above are selected.

The second embodiment of the present invention comprises a method for screening prophylactic and/or therapeutic agents for neurodegenerative diseases, comprising:
(i) the step of exposing neural stem cells to a test substance and measuring the expression of any of the genes listed in Table 2 above or the expression and/or activity of the proteins derived from the genes above; and
(ii) the step of selecting a test substance which exhibits an expression promoting effect on the genes above or; an expression promoting effect or activating effect on a protein derived from the genes above, based on the results of step (i) above.

The aforementioned-description of the first embodiment above can apply to the neural stem cells and the test substances above.

As a method for measuring the expression of any of the genes listed in Table 2 above or the expression and/or activity of the proteins derived from the genes above, any conventionally known method for measuring gene expression, protein expression, or protein activity in various cells can be used. The method can include, for example, measuring the expression of each gene, the expression and/or activity of each protein in the presence and absence of the test substance, and assessing the measured result based on differences between the presence and absence of the test substance or on the ratio of the two, and the like. Using these methods and the like, test substances which exhibit an expression promoting effect on the genes above or; an expression promoting effect or activity enhancing effect on a protein derived from the above genes are selected.

The substances selected by the screening method above can efficiently activate neural stem cells, promote their proliferation, and produce a large number of neurons. Therefore, it is possible to restore neural stem cells in the aged brain to a state seen in adolescence or an even younger period, produce a large number of neurons, and potentially improve memory and learning abilities. In addition, therapeutic effects on neurodegenerative diseases such as Alzheimer's disease are also expected, as the endogenous neural stem cells can be efficiently activated to produce a large number of neurons, thereby improving memory and learning abilities.

### EXAMPLES

The present disclosure is described specifically by way of the examples below, but the present disclosure is not to be construed as being limited to these examples.

The materials and methods used in the examples were as follows.

### <Animals>

5XFAD mice, model mice of Alzheimer's disease (Oakley et al., 2006), were obtained from Jackson Laboratory. In simple terms, 5XFAD mice carry a double transgenic construct of mutant human amyloid precursor protein (APP) (Swedish mutation: K670N, M671L; Florida mutation: I716V; London mutation: V717I) and presenilin 1 (PS1) (M146L; L286V) under the control of the mouse Thy1 promoter (Tg6799 line). The 5×FAD line (B6/SJL genetic background) was obtained from Jackson Laboratory and backcrossed with C57BL/6J wild-type (WT) mice for more than two generations. WT littermate mice were used as control animals. All mice were handled in accordance with the Guidelines for the Management and Use of Experimental Animals at Kyoto University. The experimental protocol was approved by the Animal Experiment Committee of Institute for Frontier Life and Medical Sciences, Kyoto University.

### <Construction of lentivirus>

The sequence of each gene was subcloned into the CSII-1.6-kb-pmHes5-mCherry-3NLS-MCS or CSII-pEFS-mCherry-3NLS-MCS plasmid to produce lentivirus. For lentivirus production, each CSII plasmid (84 *µ*g), gag-pol plasmid (psPAX2, 36.5 *µ*g), and VSV-G plasmid (pMD2.G, 11.7 *µ*g) were co-transfected into HEK293 cells in five 15-cm dishes using 860 *µ*L of 1 mg/ml polyethyleneimine-MAX (Polysciences). Cells were cultured in OptiPRO serum-free medium (Gibco) containing 10 *µ*M Forskolin, 1 mM sodium pyruvate, and GlutaMax (Thermo Fisher Scientific Inc.), and the medium was collected after 3 days. A total of 100 mL of medium was concentrated into 100 *µ*L of PBS by centrifugation at 8,000 ×g for 12 hours and at 13,000 ×g for 4 hours. The lentiviral titer was measured by infecting cultured NSCs, and typically a titer of 2×10⁷ units/ml was obtained. A miR-E-based lentiviral vector was used for gene expression knockdown. The sequences shown in the table below were used for knockdown. A scrambled sequence was used as a negative control.

**[Table 3]**

| SEQ ID NO: | Gene Name | Sequence used for knockdown |
|---|---|---|
| 1 | Maml2 | TTTATCTACTAGATGACAGTCT |
| 2 | Ptgds | TCTTGTTGAAAGTTGGGCTGCA |
| 3 | Ell3 | TTCATCTTCATCTTGGCCCCAG |
| 4 | Prkag2 | TTTATGAAATCTGTAATCGTGA |
| 5 | Gfra2 | TTGATCACCTTTTTACTCCCTG |
| 6 | Itpk1 | TTAATGATGATGTCAATTCCAA |
| 7 | Mical3 | TTTTCAATAACAACCACCTTGG |
| 8 | Rpl29 | TTCGTATCTTTGCGACCGGGGT |

### <Lentiviral infection in mice>

For in vivo screening and induction of neurogenesis, 1 *µ*L of lentivirus (3.75 × 10⁵ U/mL) was stereotaxically delivered to both sides of the dentate gyrus at coordinates from Bregma: anterior-posterior = -2.0 mm, lateral = ±1.4 mm, ventral = 2.0 mm, at a flow rate of 0.125 *µ*L/min. Next, the brain sections were examined immunohistochemically.

### <Immunohistochemical analysis>

For immunohistochemical analysis of brain tissue, mice were perfused through the heart using PBS containing 4% paraformaldehyde. Afterwards, the brain was post-fixed in PBS containing 4% paraformaldehyde, then incubated continuously overnight in PBS containing 30% sucrose for cryoprotection, embedded, and frozen in OCT (Tissue TEK). Consecutive 50 µm thick coronal frozen sections were obtained using a cryostat. Primary antibodies used were rabbit anti-Doublecortin (1:500; Cell Signaling Technology 14802), rabbit anti-MCM2 (1:500; Abcam ab4461), rat anti-mCherry (1:500; Invitrogen M11217), and mouse anti-*β*-Amyloid (clone 6E10; 1:500; BioLegend 9320). The primary antibody was incubated for two nights, and the secondary antibody was incubated for one night.

### <Quantification of marker cells>

DG-SGZ was analyzed in each experiment with three or more mice. The DG region of each mouse was evaluated for every seventh section. Immunostaining was performed on all sections except the ones damaged for labeling. After that, the z-stack images were captured using a confocal microscope (LSM780 or LSM980) with a 10x or 20x objective. The stained cells were quantified using ImageJ and indicated as the number of cells per mm3 of DG-SGZ in each image.

### <Quantification of amyloid-β (Aβ) deposition>

Brain sections from each mouse were labeled with a mouse anti-amyloid *β* primary antibody (clone 6E10; 1:500; BioLegend 9320) every seven sections and detected with a fluorescently labeled secondary antibody. After immunostaining, z-stack images were obtained using a confocal microscope (LSM780 or LSM980) with 10x or 20x objectives. The number of A*β* plaques was quantified using ImageJ after appropriate thresholding and noise despeckle processing. The results were indicated as the plaque coverage area per mm3 of the DG-SGZ in each image.

### <Preparation of Dentate Gyrus Tissue from Lentivirus-Injected 5XFAD Mice and Transcriptome Analysis>

Four weeks after infecting 5-month-old 5XFAD mice with a lentivirus, total RNA was isolated from DG tissue using the Rneasy Mini Kit (Qiagen). RNA quality was evaluated using an RNA Bioanalyzer, and it was confirmed that the RNA Integrity Number (RIN) was 9 or higher in all samples. The sequencing library was prepared using the NEBNext Ultra II Directional RNA Library Prep Kit for Illumina (NEB). Paired-end sequencing was performed using HiSeq X (Illumina). The cDNA sequences were aligned to the mouse reference genome mm10, counted using STAR, and normalized with edgeR using the TPM normalization method. Samples which showed abnormal values were excluded from the analysis. Gene expression analysis and GO enrichment analysis were performed using edgeR and Metascape. The ligand-receptor pair list was obtained from CellTalkDB (Shao et al. 2021).

### <Statistical analysis>

The data were shown as the mean ± SEM. Statistical analysis was performed using R software. Statistical differences were subjected to post hoc comparison tests using one-way ANOVA, two-way ANOVA, the Tukey-Kramer method, and the Bonferroni method. P-value of < 0.05 was considered statistically significant.

### 1. Enhancement of neurogenesis and suppression of Aβ deposition by iPaD

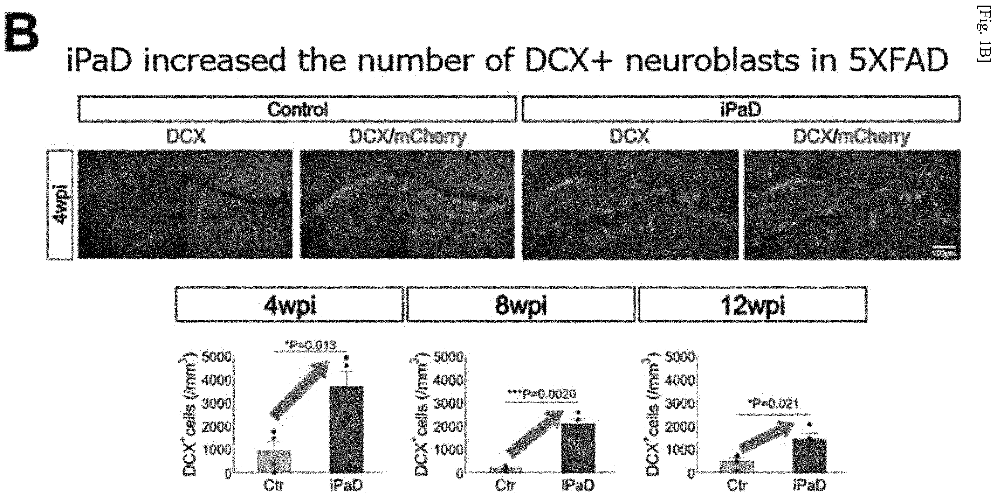

To clarify the influences of activation of neurogenesis on Alzheimer's disease, either a control lentivirus or an iPaD lentivirus (combination of Plagl2 overexpression and Dyrk1a knockdown: see Non-Patent Literature 6 for details) was injected into the hippocampal dentate gyrus of 5-month-old 5XFAD mice under the control of the Hes5 promoter, which is activated in NSCs (neural stem cells). The hippocampal region was examined after a certain period (4, 8, or 12 weeks post lentiviral infection (wpi)). NSCs infected with lentivirus and their progeny cells were mCherry+ (Fig. 1). In control lentivirus-injected 5XFAD mice, activated NSCs (MCM2+, Fig. 1A, control) or DCX+ neuroblasts (Fig. 1B, control) were very few at all stages of examination, indicating that neurogenesis was significantly inhibited. In contrast, in iPaD lentivirus-injected 5XFAD mice, activated NSCs (MCM2+, Fig. 1A, iPaD) and DCX+ neuroblasts (Fig. 1B, iPaD) were abundant at all stages of examination, indicating that NSCs were significantly activated and neurogenesis was continuously enhanced.

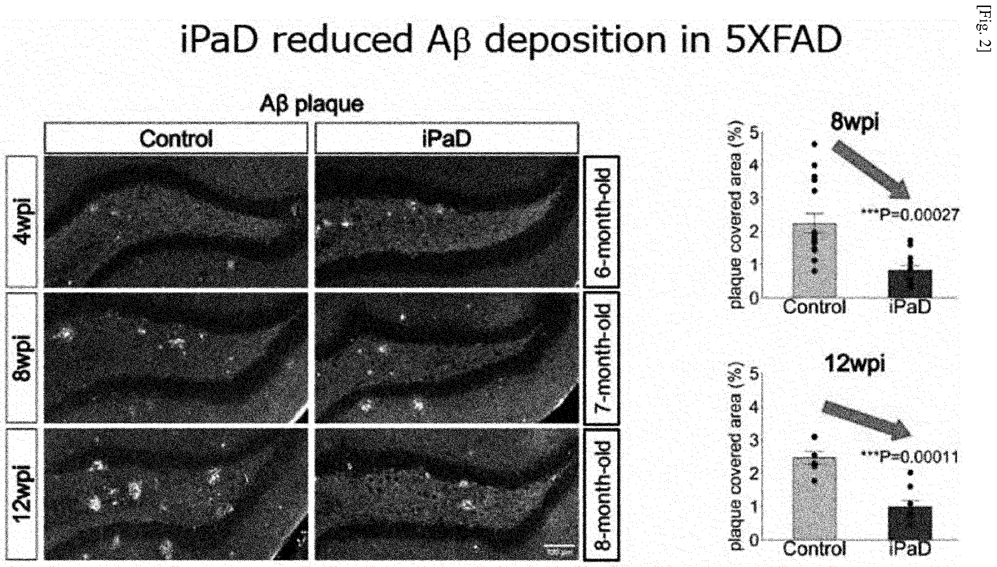

The effects of iPaD treatment on A*β* pathology were also examined. In control lentivirus-injected 5XFAD mice, A*β* deposition increased with aging (Fig. 2, control). In contrast, in iPaD lentivirus-injected 5XFAD mice, A*β* deposition did not increase with aging (Fig. 2, iPaD). From the above, it was suggested that iPaD treatment efficiently activates NSCs, promotes neurogenesis, and reduces A*β* deposition. In addition, the combination of Plagl2 overexpression and Dyrk1 a knockdown was named by the present inventors as "iPaD (inducing Plagl2 and anti-Dyrk1a)".

### 2. Downstream events of iPaD treatment

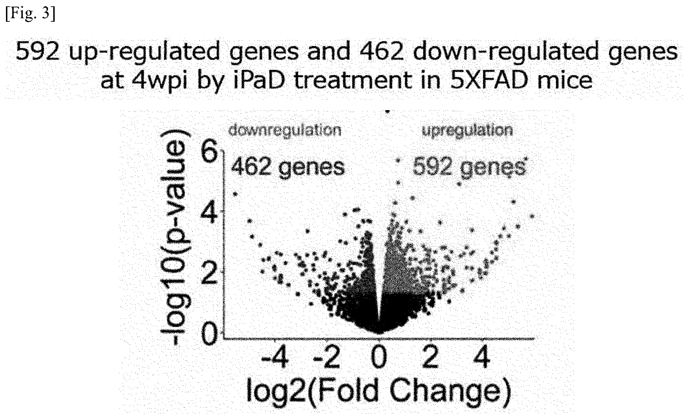

In order to understand the mechanism by which NSCs improve the phenotype of Alzheimer's disease through iPaD treatment, iPaD lentivirus was injected into the hippocampal dentate gyrus of 5-month-old 5XFAD mice, and the hippocampus was isolated for RNA-seq analysis four weeks later. As a result, iPaD-treated NSCs significantly upregulated 592 genes and downregulated 462 genes throughout the hippocampus of 5XFAD mice (Fig. 3, p-value < 0.05).

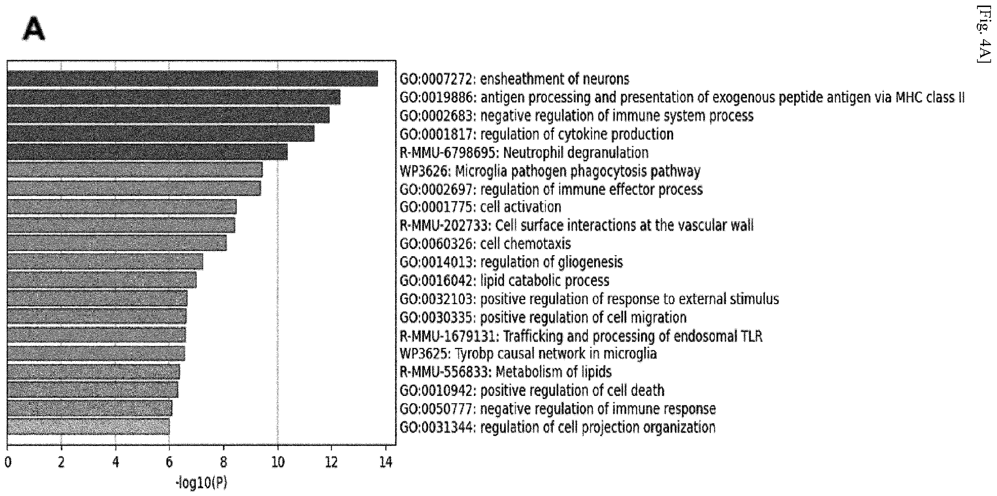

Gene ontology (GO) analysis of upregulated genes revealed that these genes included those involved in myelination, immunity, cytokines, and microglia (Fig. 4A). From these genes, ligand-receptor pairs published in CellTalkDB (http://tcm.zju.edu.cn/celltalkdb/) were extracted, and it was confirmed that ligand-receptor pairs such as Apoe-Abca1/Trem2, Il33-Il1rl1, Ccl3-Ccr5, C3/Icam1-Itgb2, and Lgals3-Mertk, shown in Table 4 below, are the ligand-receptor pairs which are upregulated in the downstream of iPaD treatment. Because these ligand-receptor pairs are known to improve the pathology of Alzheimer's disease (Salta E. et al., 2023. Adult hippocampal neurogenesis in Alzheimer's disease: a road map to clinical relevance. Cell Stem Cell 30: 120-136), these results suggest that iPaD treatment improves the pathology of Alzheimer's disease by activating these ligand-receptor pairs.

**[Table 4]**

| ID | Ligand/ Receptor | Official Symbol | Gene Name | Ensembl Gene ID | Transcript ID |
|---|---|---|---|---|---|
| 1 | Ligand | Apoe | apolipoprotein E | ENSMUSG00000002985 | ENSMUST00000174064 |
| | Receptor | Abca 1 | ATP-binding cassette, sub-family A member 1 | ENSMUSG00000015243 | ENSMUST00000030010 |
| 2 | Ligand | Il33 | interleukin 33 | ENSMUSG00000024810 | ENSMUST00000025724 |
| | Receptor | Il1rl1 | interleukin 1 receptor-like 1 | ENSMUSG00000026069 | ENSMUST00000097772 |
| 3 | Ligand | Adam17 | a disintegrin and metallopeptidase domain 17 | ENSMUSG00000052593 | ENSMUST00000101551 |
| | Receptor | Notch2 | notch 2 | ENSMUSG00000027878 | ENSMUST00000079812 |
| 4 | Ligand | Ccl3 | C-C motif chemokine ligand 3 | ENSMUSG00000000982 | ENSMUST00000001008 |
| | Receptor | Ccr5 | C-C motif chemokine receptor 5 | ENSMUSG00000079227 | ENSMUST00000111442 |
| 5 | Ligand | Apoe | apolipoprotein E | ENSMUSG00000002985 | ENSMUST00000174064 |
| | Receptor | Trem2 | triggering receptor expressed on myeloid cells 2 | ENSMUSG00000023992 | ENSMUST00000113237 |
| 6 | Ligand | C3 | complement component 3 | ENSMUSG00000024164 | ENSMUST00000024988 |
| | Receptor | Itgb2 | integrin beta 2 | ENSMUSG00000000290 | ENSMUST00000000299 |
| 7 | Ligand | Icam1 | intercellular adhesion molecule 1 | ENSMUSG00000037405 | ENSMUST00000086399 |
| | Receptor | Itgb2 | integrin beta 2 | ENSMUSG00000000290 | ENSMUST00000000299 |
| 8 | Ligand | Adam17 | a disintegrin and metallopeptidase domain 17 | ENSMUSG00000052593 | ENSMUST00000101551 |
| | Receptor | Tnfrsf1a | tumor necrosis factor receptor superfamily, member 1a | ENSMUSG00000030341 | ENSMUST00000032491 |
| 9 | Ligand | Pros1 | protein S (alpha) | ENSMUSG00000022912 | ENSMUST00000023629 |
| | Receptor | Mertk | MER proto-oncogene tyrosine kinase | ENSMUSG00000014361 | ENSMUST00000014505 |
| 10 | Ligand | Lgals3 | lectin, galactose binding, soluble 3 | ENSMUSG00000050335 | ENSMUST00000142734 |
| | Receptor | Mertk | MER proto-oncogene tyrosine kinase | ENSMUSG00000014361 | ENSMUST00000014505 |

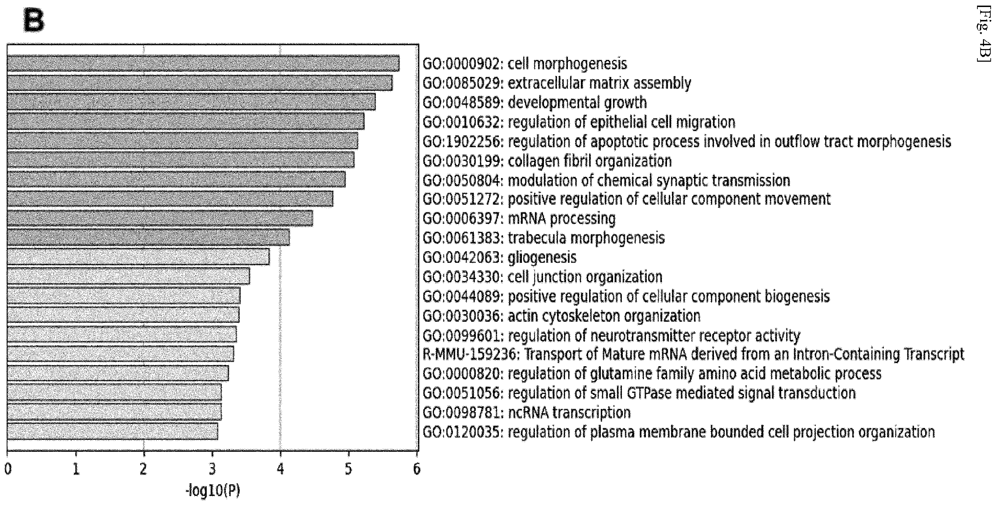

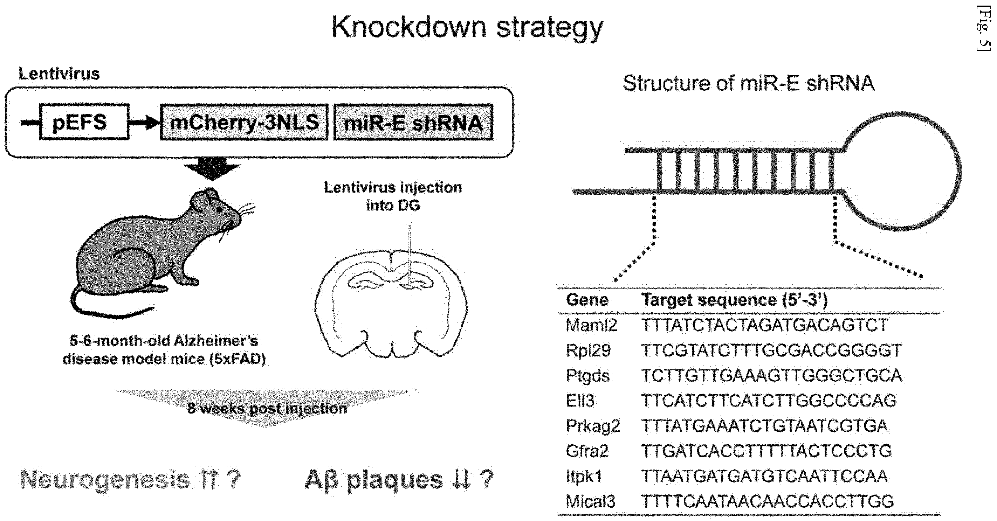

In the GO term analysis of downregulated genes, genes involved in cell morphogenesis, extracellular matrix (ECM), and developmental growth were included (Fig. 4B). Some ECM proteins are known to be involved in the pathology of Alzheimer's disease (Sun et al., 2021, Role of the extracellular matrix in Alzheimer's disease. Front Aging Neurosci 13: 707466), but the functional significance of most other genes has not been analyzed. Therefore, the pathology of Alzheimer's disease was examined using the miR-E-based shRNA system to determine whether the knockdown of these genes could improve the pathology (Fellmann et al., 2013, An optimized microRNA backbone for effective single-copy RNAi. Cell Rep 5: 1704-1713). This knockdown strategy is shown in Fig. 5.

3. Increase in neurogenesis and reduction in A*β* deposition due to knockdown of Prkag2, Gfra2, Rp129, and Maml2

The table below shows the list of genes whose expressions were most significantly decreased by iPaD treatment in the hippocampus of 5XFAD mice.

**[Table 5]**

| ID | Official Symbol | Gene Name | Ensembl Gene ID | Transcript ID |
|---|---|---|---|---|
| 1 | Maml2 | mastermind like transcriptional coactivator 2 | ENSMUSG00000031925 | ENSMUST00000159294 |
| 2 | Rpl29 | ribosomal protein L29 | ENSMUSG00000048758 | ENSMUST00000150576 |
| 3 | Ptgds | prostaglandin D2 synthase (brain) | ENSMUSG00000015090 | ENSMUST00000015234 |
| 4 | Ell3 | elongation factor RNA polymerase II-like 3 | ENSMUSG00000027246 | ENSMUST00000028679 |
| 5 | Grik3 | glutamate receptor, ionotropic, kainate 3 | ENSMUSG00000001985 | ENSMUST00000030676 |
| 6 | Col5a1 | collagen, type V, alpha 1 | ENSMUSG00000026837 | ENSMUST00000028280 |
| 7 | Prkag2 | protein kinase, AMP-activated, gamma 2 non-catalytic subunit | ENSMUSG00000028944 | ENSMUST00000030784 |
| 8 | Cspp1 | centrosome and spindle pole associated protein 1 | ENSMUSG00000056763 | ENSMUST00000071087 |
| 9 | A830036E02Rik | RIKEN cDNA A830036E02 gene | ENSMUSG00000084890 | ENSMUST00000139095 |
| 10 | 4930474M22Rik | RIKEN cDNA 4930474M22 gene | ENSMUSG00000117328 | ENSMUST00000233310 |
| 11 | Gfra2 | glial cell line derived neurotrophic factor family receptor alpha 2 | ENSMUSG00000022103 | ENSMUST00000022699 |
| 12 | Itpk1 | inositol 1,3,4-triphosphate 5/6 kinase | ENSMUSG00000057963 | ENSMUST00000046518 |
| 13 | Sh3rf3 | SH3 domain containing ring finger 3 | ENSMUSG00000037990 | ENSMUST00000153031 |
| 14 | Mical3 | microtubule associated monooxygenase, calponin and LIM domain containing 3 | ENSMUSG00000051586 | ENSMUST00000238968 |
| 15 | Abcc8 | ATP-binding cassette, sub-family C member 8 | ENSMUSG00000040136 | ENSMUST00000033123 |
| 16 | Flnb | filamin, beta | ENSMUSG00000025278 | ENSMUST00000052678 |
| 17 | Lime1 | Lck interacting transmembrane adaptor 1 | ENSMUSG00000090077 | ENSMUST00000048077 |
| 18 | Zswim4 | zinc finger SWIM-type containing 4 | ENSMUSG00000035671 | ENSMUST00000039480 |
| 19 | Cdc42bpg | CDC42 binding protein kinase gamma | ENSMUSG00000024769 | ENSMUST00000025681 |
| 20 | Kcnh1 | potassium voltage-gated channel, subfamily H (eag-related), member 1 | ENSMUSG00000058248 | ENSMUST00000078470 |
| 21 | Col5a1 | collagen, type V, alpha 1 | ENSMUSG00000026837 | ENSMUST00000028280 |
| 22 | Adamts17 | ADAM metallopeptidase with thrombospondin type 1 motif 17 | ENSMUSG00000058145 | ENSMUST00000098382 |
| 23 | Smoc2 | SPARC related modular calcium binding 2 | ENSMUSG00000023886 | ENSMUST00000233344 |
| 24 | Colla1 | collagen, type I, alpha 1 | ENSMUSG00000001506 | ENSMUST00000001547 |
| 25 | Wnt6 | wingless-type MMTV integration site family, member 6 | ENSMUSG00000033227 | ENSMUST00000006716 |
| 26 | Col1a2 | collagen, type I, alpha 2 | ENSMUSG00000029661 | ENSMUST00000031668 |
| 27 | Ccbe1 | collagen and calcium binding EGF domains 1 | ENSMUSG00000046318 | |
| 28 | Tgfbr3 | transforming growth factor, beta receptor III | ENSMUSG00000029287 | ENSMUST00000031224 |
| 29 | Mgp | matrix Gla protein | ENSMUSG00000030218 | ENSMUST00000032342 |
| 30 | Dmp1 | dentin matrix protein 1 | ENSMUSG00000029307 | ENSMUST00000066708 |
| 31 | Tnxb | tenascin XB | ENSMUSG00000033327 | ENSMUST00000238967 |
| 32 | Pxdn | peroxidasin | ENSMUSG00000020674 | ENSMUST00000122328 |
| 33 | Lamb1 | laminin B1 | ENSMUSG00000002900 | ENSMUST00000239496 |
| 34 | Omd | osteomodulin | ENSMUSG00000048368 | ENSMUST00000221170 |
| 35 | Rtn4rl2 | reticulon 4 receptor-like 2 | ENSMUSG00000050896 | ENSMUST00000151799 |
| 36 | Ltbp4 | latent transforming growth factor beta binding protein 4 | ENSMUSG00000040488 | ENSMUST00000038618 |
| 37 | Tgfb2 | transforming growth factor, beta 2 | ENSMUSG00000039239 | ENSMUST00000195201 |
| 38 | Fbln1 | fibulin 1 | ENSMUSG00000006369 | ENSMUST00000057410 |

From the table above, Maml2, Rp129, Ptgds, Ell3, Prkag2, Cspp1, Gfra2, Itpk1, and Mical3, which were widely expressed but most significantly suppressed by iPaD treatment, were selected to examine the overall knockdown effect on the A*β* pathology. Using a lentivirus, along with mCherry, the expressions of miR-E-based shRNAs of each gene (SEQ ID NOs: 1 to 8 in Table 3) in the hippocampus of 5XFAD mice were induced under the control of ubiquitous elongation factor promoter, and their effects were examined 8 weeks later (Fig. 5).

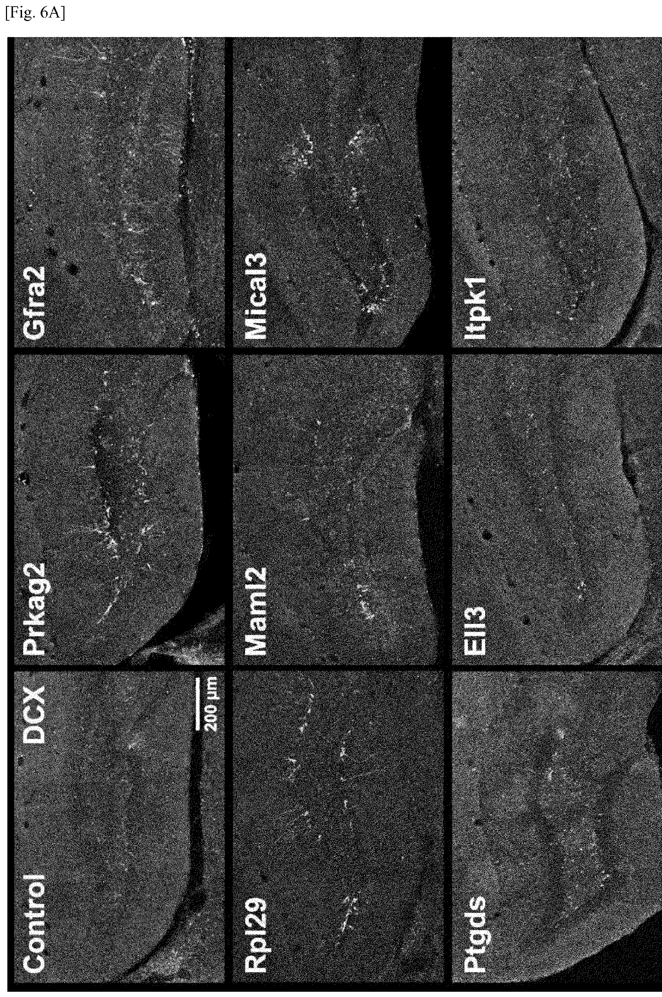

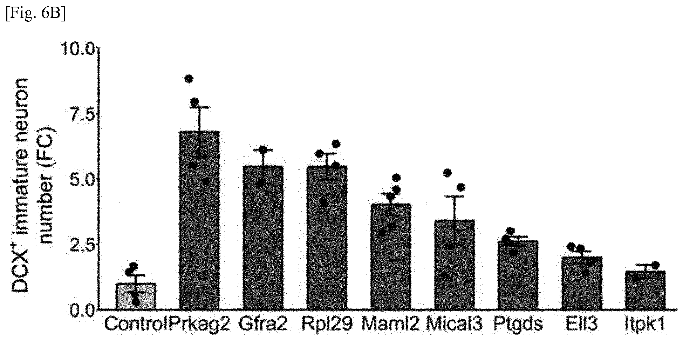

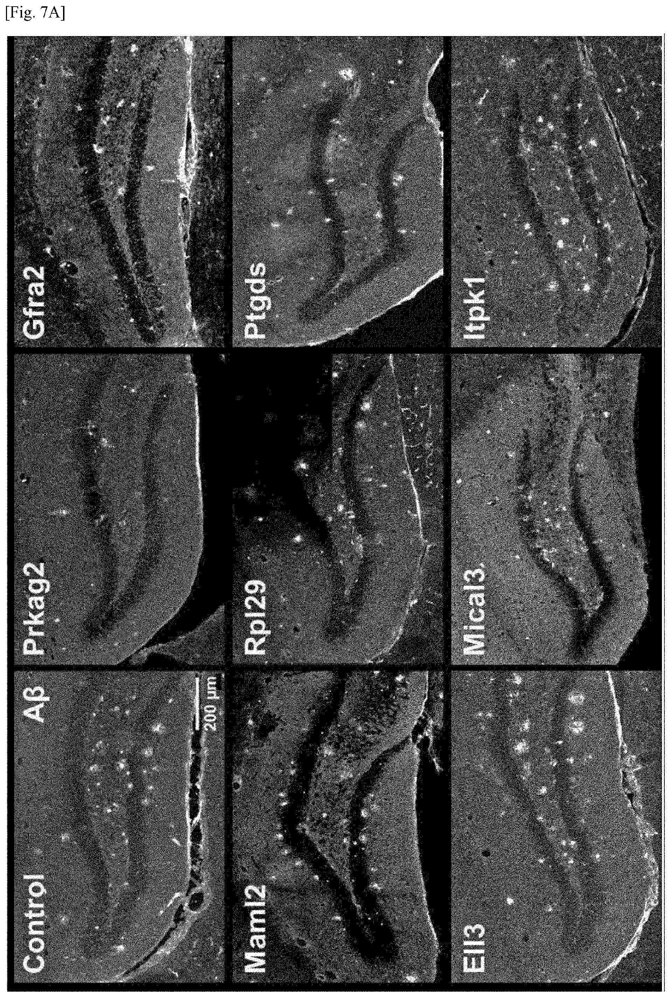

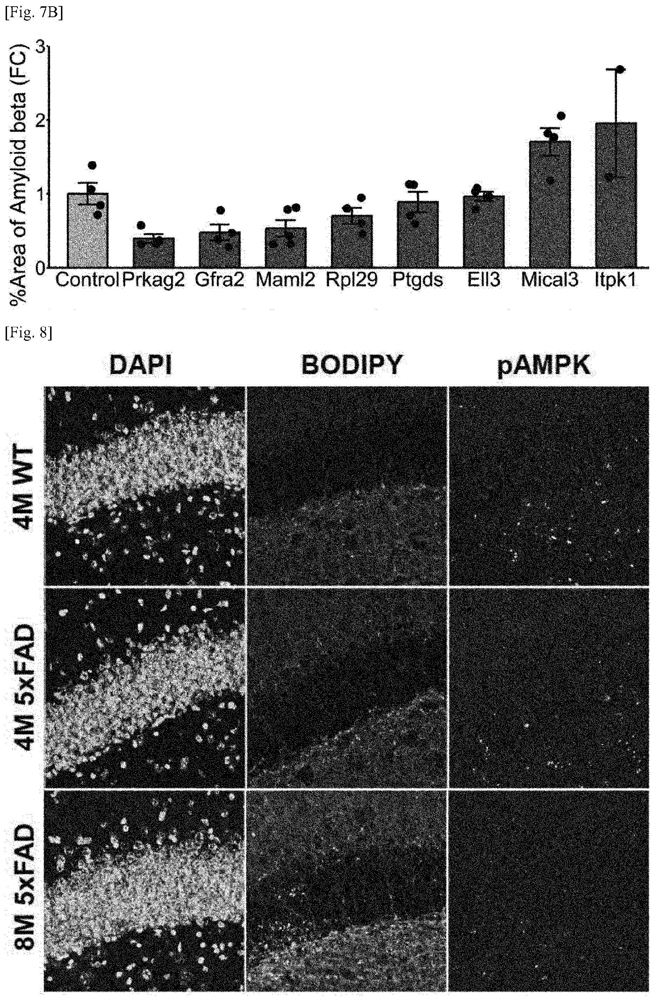

Among the examined genes above, knockdown of Prkag2, which encodes the non-catalytic subunit y2 of protein kinase AMP-activated, Gfra2, which encodes the glial cell line-derived neurotrophic factor family receptor α2, and Rp129, which encodes a protein component of the ribosome, more effectively increased the number of DCX+ neuroblasts (Figs. 6A and 6B) and suppressed A*β* deposition (Figs. 7A and 7B). The knockdown of Maml2, which encodes a co-activator of Notch signaling, similarly increased the number of DCX+ neuroblasts (Figs. 6A and 6B) and suppressed A*β* deposition (Figs. 7A and 7B), but the effect was lower than that of Prkag2. These results suggest that the knockdown of Prkag2, Gfra2, Rp129, and Maml2 effectively activates neurogenesis and improves A*β* pathology, suggesting that these genes can serve as new targets for Alzheimer's disease therapy.

As described above, many genes involved in cell morphogenesis, extracellular matrix (ECM), and developmental growth were downregulated by iPaD treatment, among which the downregulation of Prkag2, Gfra2, Rp129, and Maml2 was found to be important for the activation of neurogenesis and the suppression of A*β* accumulation.

### 4. Elucidation of the mechanism of action by which knockdown of Prkag2 increases neurogenesis and reduces Aβ deposition

Prkag2, which showed the most remarkable increase in neurogenesis and reduction effect in A*β* deposition due to knockdown, was focused, and its mechanism of action was investigated. Prkag2 is one of the subunits which compose AMP-activated protein kinase (AMPK) (y subunit 2). The AMPK signaling is known to activate autophagy to degrade amyloid *β* deposition, as well as reduce lipid droplets and activate the phagocytic ability of microglia. AMPK signaling is considered, by these actions, to improve the pathology of Alzheimer's disease. When measuring the amount of activated AMPK (phosphorylation of Thr172 on the AMPKα subunit = pAMPK), 4-month-old 5xFAD mice had a comparable number of pAMPK-positive cells to wild-type mice, and the amount of lipid droplets (BODIPY+) was also similar (Fig. 8, 4M WT and 4M 5xFAD). However, by eight months of age, pAMPK-positive cells were remarkably decreased in 5xFAD mice, while conversely, the amount of lipid droplets increased (Fig. 8, 8M 5xFAD). Also, at this period, amyloid *β* deposition was remarkably increased in 5xFAD mice. Therefore, it was suggested that a decrease in AMPK signaling leads to worsen the pathology of Alzheimer's disease, such as an increase in amyloid *β* deposition and lipid droplets.

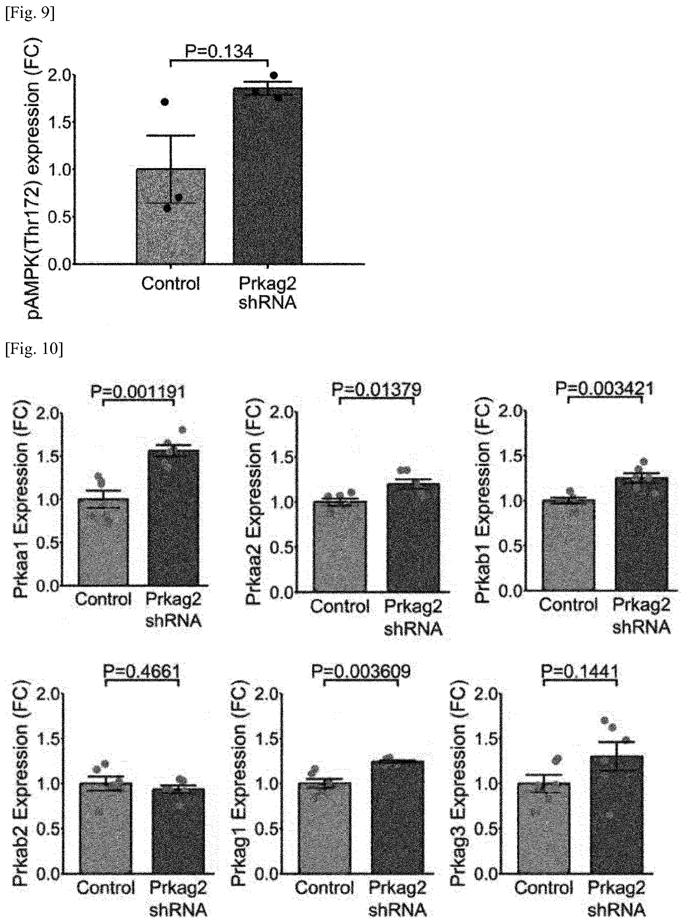

Next, the effect of Prkag2 knockdown was analyzed using a neural stem cell culture system, to find out that AMPK activity increased (Fig. 9). In order to clarify the reason why AMPK activity increased due to Prkag2 knockdown, changes in the expression of other AMPK subunits were examined using the qPCR method. As a result, it was found that Prkag2 knockdown significantly increased the expression of many subunits (the catalytic subunits Prkaa1 and Prkaa2, the structural subunit Prkab1, and the regulatory subunit Prkkag1) (Fig. 10), and it was considered that this led to an increase in AMPK activity. Therefore, it was considered that Prkag2 knockdown improves the pathology of Alzheimer's disease by activating AMPK signaling.

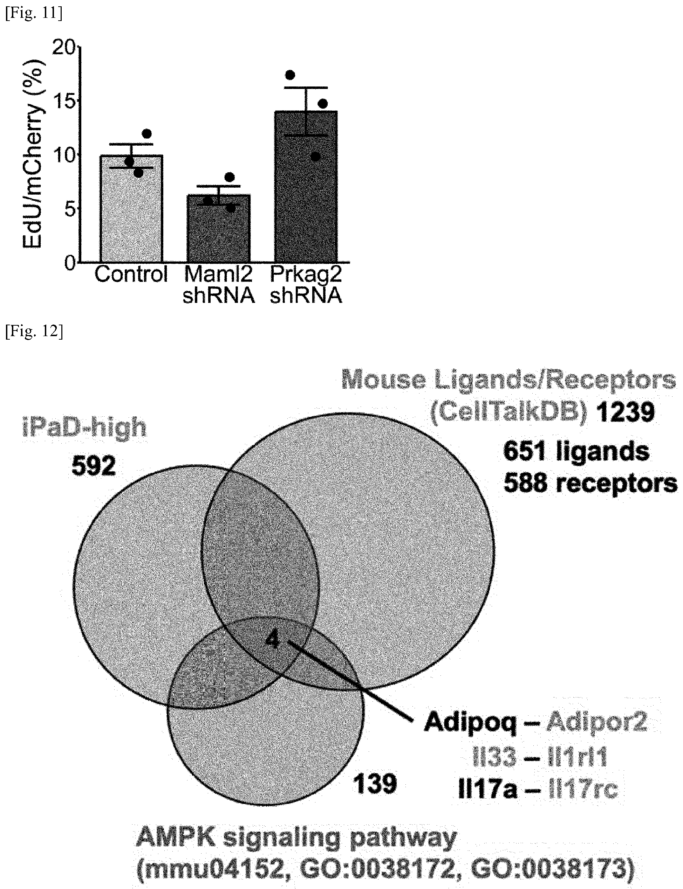

Furthermore, the effect of Prkag2 knockdown on the proliferation of cultured neural stem cells was examined, to find out that there was no significant difference, but that Prkag2 knockdown tended to increase EdU incorporation (Fig. 11). Therefore, it was suggested that Prkag2 knockdown acts directly on neural stem cells and activates their proliferative ability. On the other hand, such an effect was not observed with Maml2 knockdown (Fig. 11).

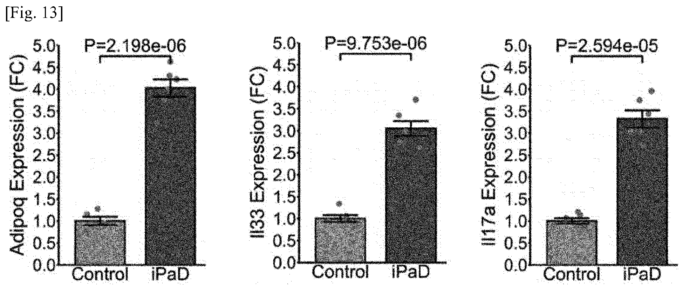

Lastly, the molecular mechanism by which activating neural stem cells by iPaD activates AMPK signaling in the surrounding region was investigated. It was suggested that neural stem cells activated by iPaD and the newborn neurons differentiated therefrom secrete AMPK-activating factors, so secretory factor-receptor pairs involved in AMPK signaling among the gene groups activated by iPaD were searched. As a result, the pairs Adipoq-Adipor2, Il33-Il1rl1, and Il17a-Il17rc were identified (Fig. 12). Furthermore, when iPaD was applied to a neural stem cell culture system, the expressions of Adipoq, Il33, and Il17a increased (Fig. 13). Therefore, it was considered that iPaD increases the production of Adipoq, Il33, and Il17a in neural stem cells, activates AMPK signaling in the surrounding region, and improves amyloid pathology.

Prkag2 is one of the regulatory subunits of AMP-activated kinase (AMPK), which regulates cellular energy metabolism. It has been reported that the expression of Prkag2 is upregulated in the postmortem brains of patients with Alzheimer's disease (Bharadwaj and Martins 2020, PRKAG2 gene expression is elevated and its protein levels are associated with increased Amyloid-β accumulation in the Alzheimer's disease brain. J Alzheimers Dis 74: 441-448), but it was unclear whether this upregulation contributes to A*β* pathology or simply responds to mitigate the pathology. In 5XFAD mice, it was found that knockdown of Prkag2 most effectively suppressed A*β* deposition, suggesting that downregulation of Prkag2 expression improves A*β* pathology not only in mice but also in humans. In addition, it was also found that knockdown of Prkag2 effectively activates neurogenesis in 5XFAD mice, but this mechanism has not yet been analyzed. It has already been reported that suppression of AMPKα 1, catalytic subunit of AMPK, alleviates cognitive impairment in Alzheimer's disease model mice (Zimmermann et.al., 2020, Brain-specific repression of AMPKα1 alleviates pathophysiology in Alzheimer's model mice. J Clin Invest 130: 3511-3527). It has also been shown that knockdown of AMPK protects model animals of amyotrophic lateral sclerosis (Lim et.al., 2012, Reduced activity of AMP-activated protein kinase protects against genetic models of motor neuron disease. J Neurosci 32: 1123-1141). These data suggest that the regulation of AMPK signaling is important for the therapy of various neurodegenerative diseases, including Alzheimer's disease.

Gfra2 is upregulated in the postmortem brains of patients with Alzheimer's disease (Haytural et.al., 2021, Insights into the changes in the proteome of Alzheimer disease elucidated by a meta-analysis. Sci Data 8: 312), but its role in Alzheimer's disease is unknown. Glial cell line-derived neurotrophic factor (GDNF), a ligand of Gfra2, has been shown to regulate axon growth and neuron survival, and neurons from Alzheimer's disease patients have been shown to lack a response to GDNF (Konishi et. al., 2014, Deficiency of GDNF receptor GFRα1 in Alzheimer's neurons results in neuronal death. J Neurosci 34: 13127-13138). In patients with Alzheimer's disease, compared with age-matched controls, serum GDNF levels were significantly lower, and cerebrospinal fluid GDNF levels were significantly higher (Straten et.al., 2009, Glial cell-line derived neurotrophic factor (GDNF) concentrations in cerebrospinal fluid and serum of patients with early Alzheimer's disease and normal controls. J Alzheimers Dis 18: 331-337.). These results suggest that the upregulation of GDNF in the cerebrospinal fluid can be an adaptive response to enhance neurotrophic support. Since GDNF selectively binds to Gfra1, a different receptor subtype, knockdown of Gfra2 can promote the interaction between GDNF and Gfra1. However, Gfra2-deficient mice exhibit behavioral and memory impairments (Voikar et al., 2004, Impaired behavioural flexibility and memory in mice lacking GDNF family receptor α2. Eur J Neurosci 20: 308-312), suggesting that a certain level of Gfra2 expression is necessary for normal brain function. Further analysis is needed to determine how knockdown of Gfra2 improves A*β* pathology. Because GDNF is upregulated by AMPK signaling (Katila et al., 2020), knockdown of Prkag2 and Gfra2 can regulate AMPK-GDNF pathway activity and improve A*β* pathology.

Maml proteins are a family of three co-activators for various transcription factors, such as the Notch signaling factor RBPj (Lin et.al., 2002, Identification of new human mastermind proteins defines a family that consists of positive regulators for notch signaling. J Biol Chem 277: 50612-50620; Wu et. al., 2002, Identification of a family of mastermind-like transcriptional coactivators for mammalian notch receptors. Mol Cell Biol 22: 7688-7700.) and myocyte enhancer factor 2C (Shen et al., 2006, The Notch coactivator, MAML1, functions as a novel coactivator for MEF2C-mediated transcription and is required for normal myogenesis. Genes Dev 20: 675-688). Therefore, knockdown of Maml2 is thought to downregulate the expression of many genes, including those involved in Notch signaling. This time, the present inventors found that knockdown of Maml2 effectively activates neurogenesis and reduces A*β* deposition. In the adult brain, the quiescence of NSCs is regulated by high expression of the Notch effector Hes1 (Sueda et.al., 2019, High Hes1 expression and resultant Ascl1 suppression regulate quiescent vs. active neural stem cells in the adult mouse brain. Genes Dev 33: 511-523), and knockdown of Maml2 can downregulate Hes1 expression, potentially leading to activation of NSCs. However, the mechanism by which knockdown of Maml2 reduces A*β* deposition has not yet been analyzed. Recently, it has been shown that Maml2 is upregulated in human Alzheimer's disease patients (Xiong et.al., 2023, Epigenomic dissection of Alzheimer's disease pinpoints causal variants and reveals epigenome erosion. Cell 186: 4422-4437), and it has been suggested that knockdown of Maml2 can be effective in reducing A*β* pathology not only in mice but also in humans.

Knockdown of Prkag2, Gfra2, Maml2, and/or Rpl29 was noted to activate adult neurogenesis, to reduce A*β* deposition, and to show that the extent of activated neurogenesis correlates well with the extent of reduction in A*β* deposition. The activation of neurogenesis reduces A*β* deposition, and the reduction of A*β* deposition activates neurogenesis, suggesting that these two phenomena can be closely related to each other. Since the activation of adult neurogenesis can improve cognitive impairment and neurodegenerative diseases associated with aging (Benraiss et.al., 2013, Mobilization of endogenous progenitor cells regenerates functionally-integrated medium spiny striopallidal projection neurons and delays disease progression in an transgenic model of Huntington's disease. Cell Stem Cell 12: 787-799.; Choi et.al., 2018, Combined adult neurogenesis and BDNF mimic exercise effects on cognition in an Alzheimer's mouse model. Science 361: eaan8821.; Diaz-Moreno et.al., 2018, Noggin rescues age-related stem cell loss in the brain of senescent mice with neurodegenerative pathology. Proc Natl Acad Sci USA 115: 11625-11630), knockdown of Prkag2, Gfra2, Maml2, and/or Rpl29 has been shown to be promising as a therapeutic strategy for various brain disorders, including Alzheimer's disease.

### INDUSTRIAL APPLICABILITY

According to the present invention, it is possible to efficiently activate neurogenesis in the adult brain and reduce the deposition of amyloid-*β* by suppressing or inhibiting the expression of the specific genes above or the proteins derived from any of these genes. Since the activation of neurogenesis and the reduction of amyloid-*β* deposition in the adult brain are thought to lead to improvements in age-related cognitive impairments and neurodegenerative diseases, the knockdown of the specific genes above such as Prkag2, Gfra2, Maml2, Rp129, or proteins derived from any of these genes is strongly suggested to be a promising therapeutic strategy for neurodegenerative diseases, including Alzheimer's disease.

## Claims

1. A prophylactic and/or therapeutic agent for neurodegenerative diseases, comprising as an active ingredient an inhibitor of any of the genes listed in Table 1 below or a protein derived from any of the genes.
**[Table 1]**
| ID | Official Symbol | Gene Name | Ensembl Gene ID | Transcript ID |
|---|---|---|---|---|
| 1 | Maml2 | mastermind like transcriptional coactivator 2 | ENSMUSG00000031925 | ENSMUST00000159294 |
| 2 | Rpl29 | ribosomal protein L29 | ENSMUSG00000048758 | ENSMUST00000150576 |
| 3 | Ptgds | prostaglandin D2 synthase (brain) | ENSMUSG00000015090 | ENSMUST00000015234 |
| 4 | Ell3 | elongation factor RNA polymerase II-like 3 | ENSMUSG00000027246 | ENSMUST00000028679 |
| 5 | Grik3 | glutamate receptor, ionotropic, kainate 3 | ENSMUSG00000001985 | ENSMUST00000030676 |
| 6 | Col5a1 | collagen, type V, alpha 1 | ENSMUSG00000026837 | ENSMUST00000028280 |
| 7 | Prkag2 | protein kinase, AMP-activated, gamma 2 non-catalytic subunit | ENSMUSG00000028944 | ENSMUST00000030784 |
| 8 | Cspp1 | centrosome and spindle pole associated protein 1 | ENSMUSG00000056763 | ENSMUST00000071087 |
| 9 | A830036E02Rik | RIKEN cDNA A830036E02 gene | ENSMUSG00000084890 | ENSMUST00000139095 |
| 10 | 4930474M22Rik | RIKEN cDNA 4930474M22 nene | ENSMUSG00000117328 | ENSMUST00000233310 |
| 11 | Gfra2 | glial cell line derived neurotrophic factor family receptor alpha 2 | ENSMUSG00000022103 | ENSMUST00000022699 |
| 12 | Itpk1 | inositol 1,3,4-triphosphate 5/6 kinase | ENSMUSG00000057963 | ENSMUST00000046518 |
| 13 | Sh3rf3 | SH3 domain containing ring finger 3 | ENSMUSG00000037990 | ENSMUST00000153031 |
| 14 | Mical3 | microtubule associated monooxygenase, calponin and LIM domain containing 3 | ENSMUSG00000051586 | ENSMUST00000238968 |
| 15 | Abcc8 | ATP-binding cassette, sub-family C member 8 | ENSMUSG00000040136 | ENSMUST00000033123 |
| 16 | Flnb | filamin, beta | ENSMUSG00000025278 | ENSMUST00000052678 |
| 17 | Lime1 | Lck interacting transmembrane adaptor 1 | ENSMUSG00000090077 | ENSMUST00000048077 |
| 18 | Zswim4 | zinc finger SWIM-type containing 4 | ENSMUSG00000035671 | ENSMUST00000039480 |
| 19 | Cdc42bpg | CDC42 binding protein kinase gamma | ENSMUSG00000024769 | ENSMUST00000025681 |
| 20 | Kcnh1 | potassium voltage-gated channel, subfamily H (eag-related), member 1 | ENSMUSG00000058248 | ENSMUST00000078470 |
| 21 | Col5a1 | collagen, type V, alpha 1 | ENSMUSG00000026837 | ENSMUST00000028280 |
| 22 | Adamts17 | ADAM metallopeptidase with thrombospondin type 1 motif 17 | ENSMUSG00000058145 | ENSMUST00000098382 |
| 23 | Smoc2 | SPARC related modular calcium binding 2 | ENSMUSG00000023886 | ENSMUST00000233344 |
| 24 | Colla1 | collagen, type I, alpha 1 | ENSMUSG00000001506 | ENSMUST00000001547 |
| 25 | Wnt6 | wingless-type MMTV integration site family, member 6 | ENSMUSG00000033227 | ENSMUST00000006716 |
| 26 | Col1a2 | collagen, type I, alpha 2 | ENSMUSG00000029661 | ENSMUST00000031668 |
| 27 | Ccbe1 | collagen and calcium binding EGF domains 1 | ENSMUSG00000046318 | |
| 28 | Tgfbr3 | transforming growth factor, beta receptor III | ENSMUSG00000029287 | ENSMUST00000031224 |
| 29 | Mgp | matrix Gla protein | ENSMUSG00000030218 | ENSMUST00000032342 |
| 30 | Dmp1 | dentin matrix protein 1 | ENSMUSG00000029307 | ENSMUST00000066708 |
| 31 | Tnxb | tenascin XB | ENSMUSG00000033327 | ENSMUST00000238967 |
| 32 | Pxdn | peroxidasin | ENSMUSG00000020674 | ENSMUST00000122328 |
| 33 | Lamb1 | laminin B1 | ENSMUSG00000002900 | ENSMUST00000239496 |
| 34 | Omd | osteomodulin | ENSMUSG00000048368 | ENSMUST00000221170 |
| 35 | Rtn4rl2 | reticulon 4 receptor-like 2 | ENSMUSG00000050896 | ENSMUST00000151799 |
| 36 | Ltbp4 | latent transforming growth factor beta binding protein 4 | ENSMUSG00000040488 | ENSMUST00000038618 |
| 37 | Tgfb2 | transforming growth factor, beta 2 | ENSMUSG00000039239 | ENSMUST00000195201 |
| 38 | Fbln1 | fibulin 1 | ENSMUSG00000006369 | ENSMUST00000057410 |

2. The prophylactic and/or therapeutic agent for neurodegenerative diseases according to Claim 1, wherein the inhibitor is;
(A) a vector containing a polynucleotide comprising a nucleic acid sequence of a miR-shRNA (microRNA adapted short hairpin RNA) against any of the genes listed in Table 1, and a promoter sequence operably linked to the nucleic acid sequence,
(B) siRNA, shRNA, or antisense oligonucleotides against any of the genes listed in Table 1,
(C) an antibody against a protein derived from any of the genes listed in Table 1, or
(D) a small molecule which has an inhibitory effect on the expression and/or function of any of the genes listed in Table 1 or of proteins derived therefrom.

3. The prophylactic and/or therapeutic agent for neurodegenerative diseases according to Claim 1 or 2, wherein the genes are mammalian genes.

4. The prophylactic and/or therapeutic agent for neurodegenerative diseases according to Claim 2 or 3, wherein the vector is lentiviral vector, adeno-associated viral vector, adenoviral vector, or plasmid vector.

5. The prophylactic and/or therapeutic agent for neurodegenerative diseases according to any one of Claims 1 to 4, which is administered by lumbar puncture.

6. The prophylactic and/or therapeutic agent for neurodegenerative diseases, comprising as an active ingredient a promoter of the expression and/or function of any of the ligand-receptor gene sets listed in Table 2 below or of a protein derived from any of the gene sets.
**[Table 2]**
| ID | Ligand/ Receptor | Official Symbol | Gene Name | Ensembl Gene ID | Transcript ID |
|---|---|---|---|---|---|
| 1 | Ligand | Apoe | apolipoprotein E | ENSMUSG00000002985 | ENSMUST00000174064 |
| | Receptor | Abca1 | ATP-binding cassette, sub-family A member 1 | ENSMUSG00000015243 | ENSMUST00000030010 |
| 2 | Ligand | Il33 | interleukin 33 | ENSMUSG00000024810 | ENSMUST00000025724 |
| | Receptor | Il1rl1 | interleukin 1 receptor-like 1 | ENSMUSG00000026069 | ENSMUST00000097772 |
| 3 | Ligand | Adam17 | a disintegrin and metallopeptidase domain 17 | ENSMUSG00000052593 | ENSMUST00000101551 |
| | Receptor | Notch2 | notch 2 | ENSMUSG00000027878 | ENSMUST00000079812 |
| 4 | Ligand | Ccl3 | C-C motif chemokine ligand 3 | ENSMUSG00000000982 | ENSMUST00000001008 |
| | Receptor | Ccr5 | C-C motif chemokine receptor 5 | ENSMUSG00000079227 | ENSMUST00000111442 |
| 5 | Ligand | Apoe | apolipoprotein E | ENSMUSG00000002985 | ENSMUST00000174064 |
| | Receptor | Trem2 | triggering receptor expressed on myeloid cells 2 | ENSMUSG00000023992 | ENSMUST00000113237 |
| 6 | Ligand | C3 | complement component 3 | ENSMUSG00000024164 | ENSMUST00000024988 |
| | Receptor | Itgb2 | integrin beta 2 | ENSMUSG00000000290 | ENSMUST00000000299 |
| 7 | Ligand | Icam1 | intercellular adhesion molecule 1 | ENSMUSG00000037405 | ENSMUST00000086399 |
| | Receptor | Itgb2 | integrin beta 2 | ENSMUSG00000000290 | ENSMUST00000000299 |
| 8 | Ligand | Adam17 | a disintegrin and metallopeptidase domain 17 | ENSMUSG00000052593 | ENSMUST00000101551 |
| | Receptor | Tnfrsf1a | tumor necrosis factor receptor superfamily, member 1a | ENSMUSG00000030341 | ENSMUST00000032491 |
| 9 | Ligand | Pros1 | protein S (alpha) | ENSMUSG00000022912 | ENSMUST00000023629 |
| | Receptor | Mertk | MER proto-oncogene tyrosine kinase | ENSMUSG00000014361 | ENSMUST00000014505 |
| 10 | Ligand | Lgals3 | lectin, galactose binding, soluble 3 | ENSMUSG00000050335 | ENSMUST00000142734 |
| | Receptor | Mertk | MER proto-oncogene tyrosine kinase | ENSMUSG00000014361 | ENSMUST00000014505 |

7. The prophylactic and/or therapeutic agent for neurodegenerative diseases according to any one of Claims 1 to 6, wherein the neurodegenerative disease is at least one selected from the group consisting of Alzheimer's disease, Parkinson's disease, Huntington's disease, and dementias caused by frontotemporal lobar degeneration.

8. The prophylactic and/or therapeutic agent for neurodegenerative diseases according to any one of Claims 1 to 6, wherein the neurodegenerative disease is at least one selected from the group consisting of Alzheimer's disease and Parkinson's disease.

9. The prophylactic and/or therapeutic agent for neurodegenerative diseases according to any one of Claims 1 to 6, wherein the neurodegenerative disease is Alzheimer's disease.
